# EUROPEAN PATENT APPLICATION

(11) **EP 4 610 251 A1**
(43) Date of publication of application: **03.09.2025**
(21) Application number: 23881205.1
(22) Date of filing: 26.05.2023
(51) Int. Cl.: C07C 237/42, C07C 255/60, A01N 37/46, A01P 7/02, A01P 7/04

(54) **HEPTAFLUOROISOPROPYL-CONTAINING BISAMIDE COMPOUND AND INTERMEDIATE COMPOUND THEREOF, AND PREPARATION METHODS THEREFOR AND USE THEREOF**

(30) Priority: 24.10.2022 CN 202211303718
(71) Applicant: Zhejiang Udragon Pesticides And Chemicals Co., Ltd., Hangzhou, Zhejiang 311106 (CN)
(72) Inventor: SHEN, Xinliang, Hangzhou, Zhejiang 311106 (CN); WU, Hualong, Hangzhou, Zhejiang 311106 (CN); CAO, Duanxiang, Hangzhou, Zhejiang 311106 (CN); CAO, Houhong, Hangzhou, Zhejiang 311106 (CN); PAN, Liying, Hangzhou, Zhejiang 311106 (CN); HUANG, Wenchao, Hangzhou, Zhejiang 311106 (CN); SHEN, Hao, Hangzhou, Zhejiang 311106 (CN); WANG, Haitao, Hangzhou, Zhejiang 311106 (CN)
(74) Representative: Metida
(86) International application number: PCT/CN2023/096577
(87) International publication number: WO 2024/087613

(57) **Abstract**

The invention provides a preparation method for bisamide compounds containing heptafluoroisopropyl groups and its intermediates, as well as the preparation method for the compounds of General Formula (I) or (II). The present invention also provides the X-ray powder diffraction characteristics of the crystalline form of the compound of General Formula (II). Compared with the prior art disclosed, the preparation process of the present invention features simple operation, mild chemical reaction conditions, environmental friendliness, hight overall yield, high purity of the final product, low production cost, and ease of industrial-scale production, thereby demonstrating significant commercial potential.

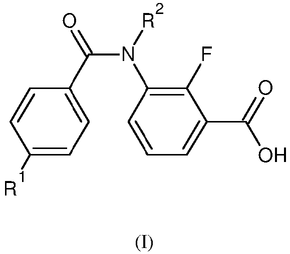

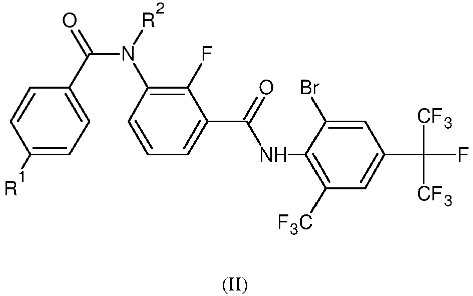

## Description

### [Technical Field]

The present invention relates to the field of insecticides, and specifically to a method for preparing a bisamide compound containing a heptafluoroisopropyl group and an intermediate thereof. The invention further relates to the X-ray powder diffraction characteristics of a crystalline form of the bisamide compound containing a heptafluoroisopropyl group.

### [Background Art]

Due to widespread and frequent use of insecticides, severe resistance issues have arisen globally, necessitating higher dosages to achieve original efficacy. In some cases, pests have become untreatable, posing challenges to agricultural production, environmental safety, and ecological balance. There is an urgent need for novel insecticides with enhanced activity, reduced dosage, and improved environmental compatibility. Research institutions and companies, both domestically and internationally, have increased investment in the development of new pesticides, leading to the emergence of insecticides with higher efficacy, lower usage, and a reduced environmental impact.

The meta-diamide insecticide Broflanilide (structural formula: 01) jointly developed by Mitsui Chemicals and BASF, has been commercialized. It is mainly used for controlling pests in fruits, vegetables, beans, cotton, corn, cereals, flowers, and non-crop applications. It is effective against lepidopteran pests, coleopteran pests, termites, ants, cockroaches, flies, and others, as detailed in Chinese Patent No. CN102119173B, "Amide Derivatives, Pest Control Agents Containing Same, and Methods of Use". Many domestic enterprises and research institutions have carried out structural optimization and improvement on this insecticide, resulting in a series of new insecticides with higher activity, lower dosage, and more environmentally friendly properties. Chinese Patent No. CN109497062 B, "Meta-Diamide Compounds, and Preparation Method and Use Thereof", has disclosed a meta-diamide compound, with the representative compound being cyclopropylflubendiamide (structural formula: 02); we have identified a bisamide compound containing a heptafluoroisopropyl group, which is represented by General Formula (II) and exhibits high insecticidal activity, with a representative structure being shown in Structural Formula 03; which provides more medication choices and schemes in agricultural and horticultural crop production. 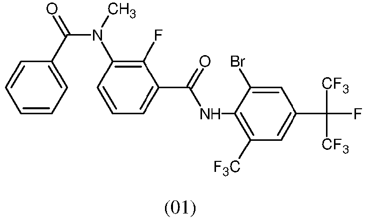 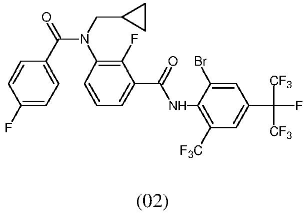 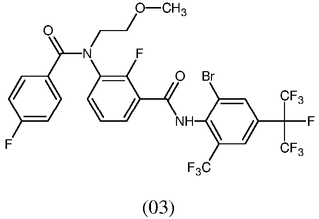 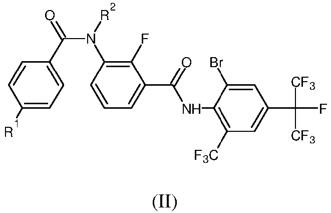 in which,
the substituent R¹ is independently selected from H, fluorine, trifluoromethyl, cyano, or nitro groups;
the substituent R² is independently selected from 2-methoxyethyl, 2-ethoxyethyl, 3-methoxypropyl, 3-ethoxypropyl, 2-n-propoxyethyl, 2-isopropoxyethyl, 2-n-butoxyethyl, cyclopropylmethyl, methyl, or ethyl groups.

However, the preparation process of the related compounds has drawbacks such as complex synthetic routes, harsh reaction conditions, poor selectivity, environmental unfriendlyness, low overall yields, difficulties in purification, and high process costs, which are not conducive to industrial-scale production and widespread application. For further details, refer to the relevant literature or patents, such as: Liu Aiping et al., "Synthesis and Biological Activity of Broflanilide", Journal of Fine Chemical Intermediates, December 2020, Vol. 50, No. 6, pp. 16-20.

### [Summary of the Invention]

In view of the deficiencies in the prior art, the objective of the present invention is to provide a method for preparing a bisamide compound containing a heptafluoroisopropyl group and its intermediates, through the optimization of the preparation process and improvement of reaction conditions. Compared with the prior art, the preparation method of the present invention features simple operation, mild chemical reaction conditions, environmental friendliness, high overall yield, high purity of the final product, low cost, and ease of industrial-scale production, thereby demonstration great potential for practical application.

To achieve the above objective, the present invention provides a method for preparing a bisamide compound containing a heptafluoroisopropyl group and intermediates thereof, which includes the method for preparing the compounds having the structures represented by General Formula (I) or (II). 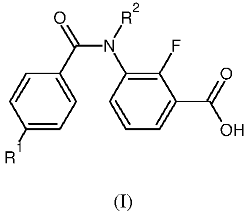
in which, the substituent R¹ is independently selected from H, fluorine, trifluoromethyl, cyano, or nitro groups;
the substituent R² is independently selected from 2-methoxyethyl, 2-ethoxyethyl, 3-methoxypropyl, 3-ethoxypropyl, 2-n-propoxyethyl, 2-isopropoxyethyl, 2-n-butoxyethyl, cyclopropylmethyl, methyl, or ethyl groups;
the process for preparing the intermediate compound of the General Formula (I) is as shown in Scheme (1) or Scheme (2): 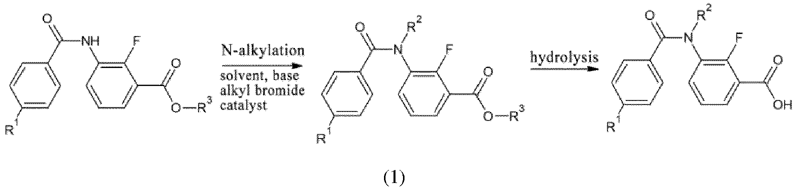 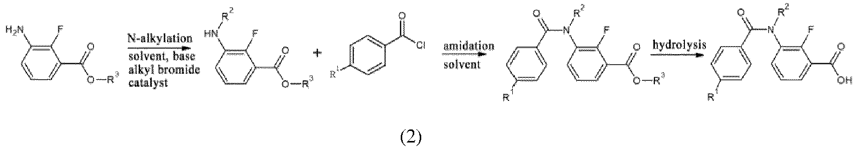 in which,
   R³ is a C1-C5 alkyl group,
   the solvent for the N-alkylation reaction is selected from N,N-dimethylformamide, N,N-dimethylacetamide, N,N-diethylformamide, N-methylpyrrolidone, 1,3-dimethyl-2-imidazolidinone, dimethyl sulfoxide, acetonitrile, tetrahydrofuran, dichloroethane, cyclohexane, methylcyclohexane, toluene, ethylbenzene, xylene, or mixtures thereof; the base for the N-alkylation reaction is selected from potassium carbonate, sodium carbonate, or sodium hydride;
   the alkyl bromide for the N-alkylation reaction is selected from 2-methoxybromoethane, 2-ethoxybromoethane, 3-methoxybromopropane, 3-ethoxybromopropane, bromomethylcyclopropane, bromomethane, or bromoethane;
   the catalyst for the N-alkylation reaction is selected from cetyltrimethylammonium chloride, benzyltriethylammonium chloride, polyethylene glycol 400-800, tetrabutylammonium chloride, tetrabutylbromide, tetrabutylhydrogen sulfate, 18-crown-6 ether, or 4-dimethylaminopyridine (DMAP), or mixtures thereof;
   the solvent for the amidation reaction is selected from N,N-dimethylformamide, N,N-dimethylacetamide, N,N-diethylformamide, N-methylpyrrolidone, 1,3-dimethyl-2-imidazolidinone, dimethyl sulfoxide, acetonitrile, tetrahydrofuran, ethyl acetate, dichloromethane, dichloroethane, cyclohexane, methylcyclohexane, toluene, ethylbenzene, xylene, or mixtures thereof.

To prepare the intermediate compound, the present invention adopts the following technical solution.

First, 2-fluoro-3-nitrobenzoic acid reacts with the corresponding alcohol in the presence of a catalyst to undergo an esterification reaction, obtaining the corresponding 2-fluoro-3-nitrobenzoate. The reaction is illustrated as follows:

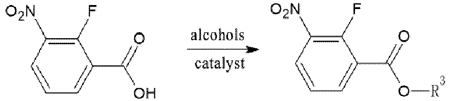

Wherein, R³ represents a C1-C5 alkyl group, preferably methyl, ethyl, isopropyl, n-butyl, or isobutyl, and particularly preferably methyl or ethyl, which is prepared through an esterification reaction with the corresponding alcohol.

The catalyst of esterification reaction can be concentrated sulfuric acid, p-toluenesulfonic acid, methylsulfonic acid, or trifluoromethylsulfonic acid. Preferably, concentrated sulfuric acid is used, with a concentration of 80-100%, and an amount of 0.5-20% by weight of the 2-fluoro-3-nitrobenzoic acid, preferably 1-5%. The alcohol used is typically a C1-C5 alcohol, preferably methanol, ethanol, isopropanol, n-butanol, or isobutanol, with methanol or ethanol being particularly preferred; the amount used is 2-10 times the weight of 2-fluoro-3-nitrobenzoic acid, preferably 3-5 times. The reaction temperature is generally 50°C to 200°C, preferably at the boiling point of the corresponding alcohol under reflux conditions. The reaction time is typically 5-48 hours, preferably 5-24 hours.

Wherein, the raw material, 2-fluoro-3-nitrobenzoic acid, is an important intermediate in pharmaceuticals and pesticides, and can be synthesized through various routes. Generally, it can be acquired through market purchase or synthesis, with a typical industrial-grade purity of over 95%. Unless otherwise specified, the present invention generally refers to obtaining the compound through market purchase, but it can also be synthesized via the following classic synthetic route:

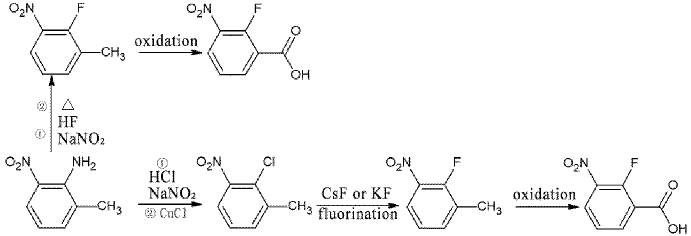

The resulting 2-fluoro-3-nitrobenzoate is then subjected to a hydrogenation reduction reaction in a solvent, in the presence of a palladium on carbon (Pd/C) or Raney nickel catalyst, to reduce the nitro group to the corresponding amino group, thereby yielding the corresponding 2-fluoro-3-aminobenzoate. The reaction is illustrated as follows:

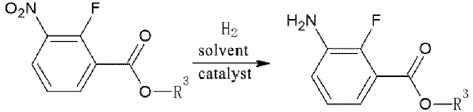

Wherein, the reaction solvent is generally selected from alcohols such as methanol, ethanol, isopropanol, and tert-butanol; or from ethers such as tetrahydrofuran, methyl tert-butyl ether, isopropyl ether, 1,4-dioxane, ethylene glycol monomethyl ether, ethylene glycol monoethyl ether, ethylene glycol dimethyl ether, and ethylene glycol diethyl ether. Alcohols are preferably used, such as methanol, ethanol, isopropanol, and tert-butanol. The amount of solvent used is typically 2-20 times the weight of 2-fluoro-3-nitrobenzoate, preferably 2-10 times. The catalyst is generally 1-5% palladium on carbon or Raney nickel, with 5% palladium on carbon being preferred. The amount of catalyst, based on dry weight, is typically 0.1-10% of the weight of 2-fluoro-3-nitrobenzoate, preferably 1-5%. From an economic perspective, the catalyst can be recycled and reused 5-10 times. Hydrogen gas should be of industrial grade with a purity of more than 99%. The reaction pressure ranges from 0 to 2 MPa, preferably from 0 to 0.5 MPa, and more preferably near atmospheric pressure.

The reaction temperature ranges from 0 to 100°C, preferably from 5 to 50 °C. The reaction time is generally until hydrogen uptake by the catalyst ceases, typically 2-24 hours.

Subsequently, the resulting 2-fluoro-3-aminobenzoate, or an equivalent commercially available compound, is reacted with a series of benzoyl chlorides in the presence of a solvent to obtain the corresponding amide intermediate. The reaction is illustrated as follows:

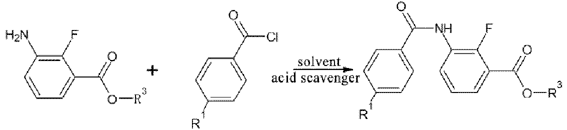

Wherein the R¹ substituent is independently selected from H, fluorine, trifluoromethyl, cyano, or nitro group; and R³ is as defined above.

The benzoyl chloride series reagents used in the amidation reaction are generally prepared by reacting the corresponding benzoic acid series raw materials with thionyl chloride in an inert solvent via an acyl chlorination reaction, followed by solvent removal. The amount used is typically 1.0-1.2 times relative to the molar amount of 2-fluoro-3-aminobenzoate, preferably 1.0-1.05 times.

In the amidation reaction, commonly used acid scavengers include triethylamine, pyridine, N,N-diisopropylethylamine, N,N-dimethylaniline, tetramethylethylenediamine, sodium bicarbonate, sodium carbonate, potassium carbonate, sodium hydroxide, potassium hydroxide, lithium hydroxide, sodium methoxide, potassium methoxide, sodium tert-butoxide, and potassium tert-butoxide. Triethylamine or pyridine is preferred. The molar amount is 0.1-5.0 times relative to the benzoyl chloride series raw materials, preferably 0.1-2.0 times for economic use. The solvents used in the amidation reaction include dichloromethane, chloroform, dichloroethane, tetrahydrofuran, dioxane, dimethyl sulfoxide, acetonitrile, toluene, xylene, N,N-dimethylformamide, N,N-dimethylacetamide, N,N-diethylformamide, N-methylpyrrolidone, and 1,3-dimethyl-2-imidazolidinone. Preferred solvents include dichloroethane, toluene, acetonitrile, N,N-dimethylformamide, N,N-dimethylacetamide, and 1,3-dimethyl-2-imidazolidinone. The amount used is typically 1-20 times the weight of the benzoyl chloride series raw materials, with 2-5 times being preferred for economical operation. The amidation reaction temperature is generally from -5°C to 150°C, preferably from 0°C to 130°C. The reaction time is typically 1-5 hours, preferably 1-2 hours.

The obtained product can be used to realize the preparation method of the intermediate compound of the present invention, and the process of the method is shown as Formula (1):

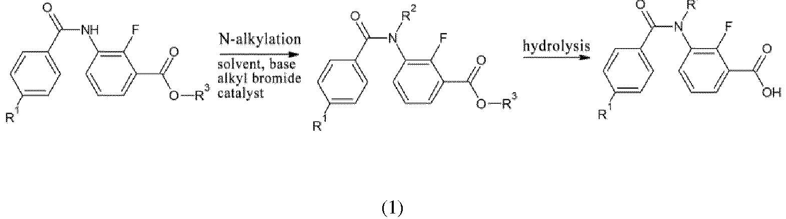

Wherein R³ represents a C1-C5 alkyl group.

The solvents used for the N-alkylation reaction can be selected from one or more mixtures of N,N-dimethylformamide, N,N-dimethylacetamide, N,N-diethylformamide, N-methylpyrrolidone, 1,3-dimethyl-2-imidazolidinone, dimethyl sulfoxide, tetrahydrofuran, acetonitrile, dichloroethane, cyclohexane, methylcyclohexane, toluene, ethylbenzene, and xylene. The preferred solvents are one or more mixtures selected from N,N-dimethylformamide, N,N-dimethylacetamide, acetonitrile, cyclohexane, methylcyclohexane, and toluene. The amount of the solvent used is generally 2-10 times the weight of the amide or 2-fluoro-3-aminobenzoate raw material, preferably 2-5 times.

The base used for the N-alkylation reaction is selected from potassium carbonate, sodium carbonate, sodium hydroxide, potassium hydroxide, and sodium hydride, preferably potassium carbonate, sodium carbonate, and sodium hydride. The amount used is typically 1-3 times relative to the molar amount of the amide or 2-fluoro-3-aminobenzoate starting material, preferably 1-2 times.

The alkyl bromide used for the N-alkylation reaction is selected from 2-methoxyethyl bromide, 2-ethoxyethyl bromide, 3-methoxypropyl bromide, 3-ethoxypropyl bromide, cyclopropylmethyl bromide, methyl bromide, and ethyl bromide. The amount used is typically 1-3 times relative to the molar amount of the amide or 2-fluoro-3-aminobenzoate starting material, preferably 1-2 times. In the present invention, alkyl bromides are employed to avoid the use of alkyl iodides. Although alkyl iodides are more reactive, they significantly increase industrial production costs and are therefore not suitable for large-scale manufacturing. Alkyl bromides, by contrast, generally exhibit lower reactivity, poor selectivity, and reduced yields. Thus, satisfactory results can only be achieved in the presence of a catalyst and under optimized reaction conditions.

For the N-alkylation reaction, the catalysts can be selected from one or more mixtures of cetyltrimethylammonium chloride, benzyltriethylammonium chloride, polyethylene glycol 400-800, tetrabutylammonium chloride, tetrabutylammonium bromide, tetrabutylammonium bisulfate, 18-crown-6 ether, or 4-dimethylaminopyridine (DMAP). Iodide salts, such as potassium iodide or sodium iodide, may also be used as catalysts; however, their catalytic effect is inferior to that of phase transfer catalysts or 4-dimethylaminopyridine (DMAP). The preferred catalysts are tetrabutylammonium chloride, tetrabutylammonium bromide, tetrabutylammonium bisulfate, 18-crown-6 ether, or 4-dimethylaminopyridine (DMAP). The amount of catalyst used is 1-10% by weight of the amide or 2-fluoro-3-aminobenzoate raw material, preferably 1-5%. The use of these catalysts in the present invention can lower the reaction temperature, improve the reaction selectivity, and enhance the product yield.

The present invention also provides another preparation method for the aforementioned intermediate compound, with the process flow illustrated by Formula (2):

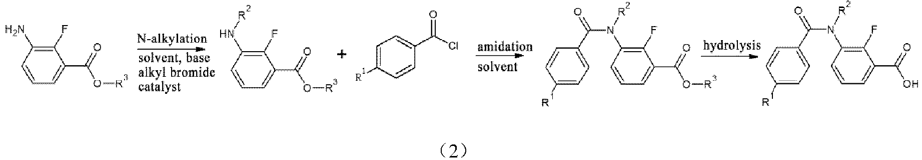

Similar to the first preparation method, R³ represents a C1-C5 alkyl group.

The solvents used for the N-alkylation reaction can be selected from one or more mixtures of N,N-dimethylformamide, N,N-dimethylacetamide, N,N-diethylformamide, N-methylpyrrolidone, 1,3-dimethyl-2-imidazolidinone, dimethyl sulfoxide, tetrahydrofuran, acetonitrile, dichloroethane, cyclohexane, methylcyclohexane, toluene, ethylbenzene, and xylene. The preferred solvents are one or more mixtures selected from N,N-dimethylformamide, N,N-dimethylacetamide, acetonitrile, cyclohexane, methylcyclohexane, and toluene. The amount of the solvent used is generally 2-10 times the weight of the amide or 2-fluoro-3-aminobenzoate raw material, preferably 2-5 times.

The base used for the N-alkylation reaction is selected from potassium carbonate, sodium carbonate, sodium hydroxide, potassium hydroxide, and sodium hydride, preferably potassium carbonate, sodium carbonate, and sodium hydride. The amount used is typically 1-3 times relative to the molar amount of the amide or 2-fluoro-3-aminobenzoate starting material, preferably 1-2 times.

The alkyl bromide used for the N-alkylation reaction is selected from 2-methoxyethyl bromide, 2-ethoxyethyl bromide, 3-methoxypropyl bromide, 3-ethoxypropyl bromide, cyclopropylmethyl bromide, methyl bromide, and ethyl bromide. The amount used is typically 1-3 times relative to the molar amount of the amide or 2-fluoro-3-aminobenzoate starting material, preferably 1-2 times. In the present invention, alkyl bromides are employed to avoid the use of alkyl iodides. Although alkyl iodides are more reactive, they significantly increase industrial production costs and are therefore not suitable for large-scale manufacturing. Alkyl bromides, by contrast, generally exhibit lower reactivity, poor selectivity, and reduced yields. Thus, satisfactory results can only be achieved in the presence of a catalyst and under optimized reaction conditions.

For the N-alkylation reaction, the catalysts can be selected from one or more mixtures of cetyltrimethylammonium chloride, benzyltriethylammonium chloride, polyethylene glycol 400-800, tetrabutylammonium chloride, tetrabutylammonium bromide, tetrabutylammonium bisulfate, 18-crown-6 ether, or 4-dimethylaminopyridine (DMAP). Iodide salts, such as potassium iodide or sodium iodide, may also be used as catalysts; however, their catalytic effect is inferior to that of phase transfer catalysts or 4-dimethylaminopyridine (DMAP). The preferred catalysts are tetrabutylammonium chloride, tetrabutylammonium bromide, tetrabutylammonium bisulfate, 18-crown-6 ether, or 4-dimethylaminopyridine (DMAP). The amount of catalyst used is 1-10% by weight of the amide or 2-fluoro-3-aminobenzoate raw material, preferably 1-5%. The use of these catalysts in the present invention can lower the reaction temperature, improve the reaction selectivity, and enhance the product yield.

The solvent for the amidation reaction is selected from one or more mixtures of N,N-dimethylformamide, N,N-dimethylacetamide, N,N-diethylformamide, N-methylpyrrolidone, 1,3-dimethyl-2-imidazolidinone, dimethyl sulfoxide, acetonitrile, tetrahydrofuran, ethyl acetate, dichloromethane, dichloroethane, cyclohexane, methylcyclohexane, toluene, ethylbenzene, and xylene. Preferred solvents are one or more of N,N-dimethylformamide, N,N-dimethylacetamide, acetonitrile, tetrahydrofuran, ethyl acetate, dichloromethane, 1,2-dichloroethane, and toluene. The amount of solvent used is 2-10 times the weight of the benzoyl chloride series raw material, preferably 2-5 times. The amidation reaction may be catalyzed by the addition of DMAP, with the amount of catalyst being 1-10% by weight of the benzoyl chloride series raw materials, preferably 1-5%. However, when no catalyst is added, the reaction yield and reaction time under the conditions of the present invention are acceptable, so the use of the catalyst is not necessary. The amidation reaction in Process Route (2) does not require the use of an acid scavenger. Under the conditions of the present invention, the conversion yield and reaction time are acceptable. The reaction temperature for the amidation in process route (2) is 0°C to 200°C, preferably 0°C to 150°C.

Among the aforementioned two preparation methods, the hydrolysis reaction is typically carried out under conventional conditions. Alcohol solvents such as methanol, ethanol, or isopropanol, or ether solvents such as 1,4-dioxane, methyl tert-butyl ether, 1,2-dimethoxyethane, or ethylene glycol diethyl ether may be used. Preferred solvents include methanol and ethanol. The amount of solvent used is typically 2-10 times the weight of the corresponding ester, preferably 2-5 times. The base is generally selected from sodium hydroxide, potassium hydroxide, sodium carbonate, and potassium carbonate, with sodium hydroxide being preferred. The base concentration is typically 1-35%, and the molar amount used is 1.1-3.0 times relative to the corresponding ester. The reaction temperature is generally 5°C to 50°C, preferably 20°C to 30°C, and the reaction time is typically 1-5 hours, preferably 1-2 hours. Among the aforementioned two preparation methods, the reaction temperature for the N-alkylation is from 50°C to 200°C, preferably from 50°C to 150°C. When the solvent forms an azeotrope with water, the reaction temperature is preferably the azeotropic temperature, and the water produced during the reaction can be removed by azeotropic distillation, which can shorten the reaction time. The reaction is typically carried out under atmospheric pressure. When the alkyl bromide is methyl bromide or ethyl bromide, due to their low boiling points and to prevent volatilization, the reaction is preferably performed in a sealed system, with a pressure of 0 to 0.2 MPa.

The reaction time for the N-alkylation is typically 2-12 hours, preferably 2-8 hours.

Furthermore, the present invention also provides a bisamide compound containing a heptafluoroisopropyl group, wherein the compound is represented by the structure of General Formula (II): 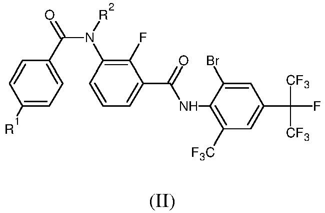 in which,
the substituent R¹ is independently selected from H, fluorine, trifluoromethyl, cyano, or nitro groups;
the substituent R² is independently selected from 2-methoxyethyl, 2-ethoxyethyl, 3-methoxypropyl, 3-ethoxypropyl, 2-n-propoxyethyl, 2-isopropoxyethyl, 2-n-butoxyethyl, cyclopropylmethyl, methyl, or ethyl groups.

The present invention provides a method for preparing a compound having the structure represented by General Formula (II). The reaction scheme is shown below:

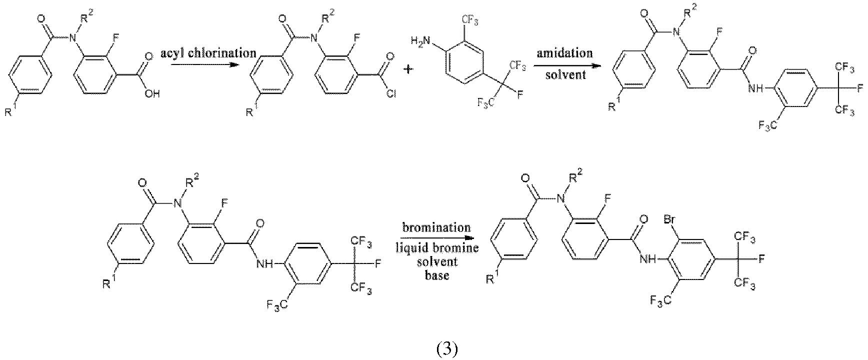

Wherein, the general method for the acyl chlorination reaction involves reacting the compound of General Formula (I) with thionyl chloride in an inert solvent, followed by solvent removal treatment before proceeding to the next reaction step. The solvents used in the acyl chlorination reaction include dichloromethane, chloroform, dichloroethane, toluene, and xylene, with dichloroethane and toluene being preferred. The reaction temperature generally ranges from 20°C to 150°C, preferably from 40°C to 100°C, for 1-5 hours, preferably 2-3 hours.

The solvents for the amidation reaction can be selected from one or more mixtures of dichloromethane, dichloroethane, cyclohexane, methylcyclohexane, toluene, ethylbenzene, xylene, tetrahydrofuran, ethyl acetate, or acetonitrile. The preferred solvents are dichloroethane, cyclohexane, methylcyclohexane, toluene, ethyl acetate, and acetonitrile. The amount of the solvent used is generally 2-10 times the weight of the raw material of General Formula (I), and preferably 3-8 times.

The amidation reaction can also utilize 4-dimethylaminopyridine (DMAP) as a catalyst, which can shorten the reaction time. However, under the preferred reaction conditions of the present invention, neither a catalyst nor an acid scavenger is required for the amidation reaction. The reaction yield and reaction time are both acceptable. Therefore, the use of these reagents is not essential for the present invention.

The other raw material for the amidation reaction is 4-heptafluoroisopropyl-2-trifluoromethylaniline, which can be obtained either by synthesis or from commercial sources. The compound can be synthesized via an alkylation reaction of o-trifluoromethylaniline with 2-bromoheptafluoropropane or 2-iodoheptafluoropropane in the presence of sodium dithionite. For details of the synthesis, refer to Chinese Patent No. CN102119173B.

The usage amount of the raw material 4-heptafluoroisopropyl-2-trifluoromethylaniline is 1.0-1.3 times the molar amount of the raw material of General Formula (I), preferably 1.0-1.05 times. In the bromination reaction, liquid bromine is used as the brominating agent. Liquid bromine is inexpensive, readily available, and the by-product bromide salt is easy to recover and reuse. Compared with N-bromosuccinimide as a brominating agent, liquid bromine has lower cost and is more environmentally friendly. The usage amount of liquid bromine is 1-2 times the molar amount of the raw material of General Formula (I), preferably1-1.5 times. The preferred addition method is dropwise into the reaction system. It can also be added dropwise under ultraviolet irradiation, but preferably proceed without the need for light.

To reduce the amount of bromine used in the bromination reaction, an oxidizing agent may be added shortly after the reaction begins, allowing the generated bromide salt to be oxidized into elemental bromine for reuse in the bromination process. This approach can lower bromine consumption, but it also increases the operational complexity of the reaction system and may lead to the formation of by-product impurities from related side reaction. Therefore, this method is not essential for the present invention. The solvent used for the bromination reaction is selected from one or more mixtures of dichloromethane, dichloroethane, methanol, ethanol, isopropanol, n-butanol, and tert-butanol. Preferred solvents include dichloroethane, methanol, ethanol, isopropanol, tert-butanol, or mixtures thereof. The amount of the solvent used is typically 2-10 times the weight of the raw material represented by General Formula (I), preferably 3-8 times.

The base used in the bromination reaction is selected from sodium hydroxide, potassium hydroxide, potassium carbonate, and sodium carbonate. Aqueous solutions of any concentration of these bases may also be used. Additionally, alcohol-based solutions such as sodium methoxide in methanol, sodium ethoxide in ethanol, sodium isopropoxide in isopropanol, and sodium tert-butoxide in tert-butanol, in any concentration are viable options. Preferred bases include solid sodium hydroxide, potassium hydroxide, or potassium carbonate. The amount of base used is typically 1-2 times the molar amount of the raw material represented by General Formula (I), preferably 1-1.5 times. The reaction temperature for the bromination is from 0°C to 100°C, preferably from 20°C to 80 °C.

In the present invention, preferably, the substituent R1 is independently selected from H or fluorine, the substituent R² is independently selected from 2-methoxyethyl, 2-ethoxyethyl, 2-n-propoxyethyl, 2-isopropoxyethyl, 2-n-butoxyethyl, cyclopropylmethyl, methyl, or ethyl.

The present invention further provides a crystalline form of bisamide compounds, wherein the compound has the structure represented by General Formula (II), and wherein R¹ is H and R² is 2-methoxyethyl, the crystalline form exhibits characteristic peaks in the X-ray powder diffraction at the following positions: 2θ = 3.521 ± 0.2°, 7.041 ± 0.2°, 8.238 ± 0.2°, 10.260 ± 0.2°, 10.578 ± 0.2°, 12.142 ± 0.2°, 12.941 ± 0.2°, 13.601 ± 0.2°, 14.922 ± 0.2°, 15.980 ± 0.2°, 17.417 ± 0.2°, 17.681 ± 0.2°, 18.321 ± 0.2°, 18.801 ± 0.2°, 19.241 ± 0.2°, 19.721 ± 0.2°, 20.420 ± 0.2°, 20.658 ± 0.2°, 21.218 ± 0.2°, 22.541 ± 0.2°, 23.379 ± 0.2°, 24.478 ± 0.2°, 24.898 ± 0.2°, 26.499 ± 0.2°, 27.420 ± 0.2°, 28.239 ± 0.2°, 29.461 ± 0.2°, 30.040 ± 0.2°, 30.839 ± 0.2°, 32.119 ± 0.2°, 32.318 ± 0.2°, 33.419 ± 0.2°, 33.741 ± 0.2°, 35.561 ± 0.2°, 37.720 ± 0.2°.

Or, wherein R¹ is fluorine and R² is 2-methoxyethyl, the crystalline form exhibits characteristic peaks in the X-ray powder diffraction at the following positions: 2θ = 7.376 ± 0.2°, 9.897 ± 0.2°, 10.940 ± 0.2°, 12.015 ± 0.2°, 12.303 ± 0.2°, 14.398 ± 0.2°, 14.578 ± 0.2°, 15.561 ± 0.2°, 15.839 ± 0.2°, 17.163 ± 0.2°, 18.257 ± 0.2°, 18.463 ± 0.2°, 18.737 ± 0.2°, 18.922 ± 0.2°, 19.219 ± 0.2°, 19.518 ± 0.2°, 19.823 ± 0.2°, 20.137 ± 0.2°, 20.379 ± 0.2°, 20.600 ± 0.2°, 20.861 ± 0.2°, 21.023 ± 0.2°, 21.722 ± 0.2°, 22.021 ± 0.2°, 22.218 ± 0.2°, 22.818 ± 0.2°, 23.999 ± 0.2°, 24.738 ± 0.2°, 25.077 ± 0.2°, 25.481 ± 0.2°, 25.860 ± 0.2°, 27.161 ± 0.2°, 27.682 ± 0.2°, 29.058 ± 0.2°, 29.323 ± 0.2°, 29.760 ± 0.2°, 29.940 ± 0.2°.

Or, wherein R¹ is fluorine and R² is 2-ethoxyethyl, the crystalline form exhibits characteristic peaks in the X-ray powder diffraction at the following positions: 2θ = 7.318 ± 0.2°, 9.697 ± 0.2°, 10.940 ± 0.2°, 11.981 ± 0.2°, 12.136 ± 0.2°, 14.142 ± 0.2°, 14.461 ± 0.2°, 15.240 ± 0.2°, 15.561 ± 0.2°, 16.304 ± 0.2°, 17.161 ± 0.2°, 17.979 ± 0.2°, 18.562 ± 0.2°, 19.138 ± 0.2°, 19.698 ± 0.2°, 20.039 ± 0.2°, 20.220 ± 0.2°, 20.442 ± 0.2°, 20.784 ± 0.2°, 20.998 ± 0.2°, 21.819 ± 0.2°, 22.039 ± 0.2°, 22.401 ± 0.2°, 23.400 ± 0.2°, 23.882 ± 0.2°, 24.383 ± 0.2°, 24.579 ± 0.2°, 25.001 ± 0.2°, 25.160 ± 0.2°, 25.459 ± 0.2°, 26.780 ± 0.2°, 26.920 ± 0.2°, 27.199 ± 0.2°, 27.378 ± 0.2°, 28.742 ± 0.2°, 29.319 ± 0.2°, 29.998 ± 0.2°, 31.339 ± 0.2°, 31.939 ± 0.2°, 32.140 ± 0.2°, 32.522 ± 0.2°, 33.479 ± 0.2°, 36.400 ± 0.2°, 43.039 ± 0.2°.

In the present invention, the aforementioned crystalline form is obtained through crystallization using a crystallization solvent, wherein the solvent is selected from one or more mixtures of methanol, ethanol, isopropanol, tetrahydrofuran, diisopropyl ether, ethylene glycol monomethyl ether, ethylene glycol monoethyl ether, ethylene glycol dimethyl ether, ethylene glycol diethyl ether, and methyl tert-butyl ether.

The bisamide compounds containing a heptafluoroisopropyl group obtained in the present invention exhibit excellent insecticidal activity. Compared with the prior art, the methods and process routes for preparing these compounds and their intermediates feature simple operational procedures, mild reaction conditions, environmental friendliness, high overall yield, high purity of the final product, low cost, and ease of industrial production, thereby demonstrating significant commercial potential.

In the present invention, operations involved in the preparation method such as distillation, vacuum distillation, filtration, drying, extraction, phase separation, crystallization, and recrystallization are conventional procedures commonly performed by those skilled in the art. These standard unit operations will not be described in detail herein. For further information, please refer to the synthesis examples.

### [Brief Description of the Drawings]

Figure 1 shows the X-ray powder diffraction (XRPD) pattern of the compound sample obtained in Synthetic Example 17.
Figure 2 shows the X-ray powder diffraction (XRPD) pattern of the compound sample obtained in Synthetic Example 18.
Figure 3 shows the X-ray powder diffraction (XRPD) pattern of the compound sample obtained in Synthetic Example 19.

### [Embodiments]

The following detailed embodiments are provided to further illustrate the technical solutions of the present invention. Those skilled in the art will understand that the embodiments are merely intended to facilitate understanding of the invention and should not be construed as limiting the scope of the invention.

Unless otherwise specified, the raw materials described in the synthetic examples of the present invention are generally obtained from commercial sources, typically with a purity specification of ≥95%, and their purity is not precisely calibrated. Unless otherwise stated, the percentage concentrations described in the synthetic examples generally refer to weight percentage concentrations. The HPLC content data represent area-normalized values without precise calibration. The yields refer to molar yields, and the yield data are likewise not precisely calibrated.

### Synthetic Preparation Example 1

In a 250mL four-neck glass reactor, 100g of methanol, 50g of 2-fluoro-3-nitrobenzoic acid, and 2.5g of 98% sulfuric acid were charged. The mixture was heated under reflux and reacted for 24 hours. After cooling to approximately 0-5 °C, the product crystallized and was filtered. The solid obtained was dried at 50 °C to yield 51g of methyl 2-fluoro-3-nitrobenzoate with an HPLC purity of 98% and a yield of 94.8%. This product was used for subsequent synthesis. Synthetic Preparation Example 2

In a 250mL four-neck glass reactor, 100g of ethanol, 50g of 2-fluoro-3-nitrobenzoic acid, and 2.5g of 98% sulfuric acid were charged. The mixture was heated under reflux and reacted for 24 hours. After cooling to approximately 0-5 °C, the product crystallized and was filtered. The solid obtained was dried at 50 °C to yield 54.5g of ethyl 2-fluoro-3-nitrobenzoate with an HPLC purity of 98% and a yield of 94.6%. This product was used for subsequent synthesis.

### Synthetic Preparation Example 3

In a 500mL hydrogenation reactor, 250g of methanol and 2g of 5% palladium on carbon (dry basis, 50% moisture content) were charged. Then, 51g of the solid methyl 2-fluoro-3-nitrobenzoate obtained from Synthetic Preparation Example 1 was added. Then the reactor was sealed. After nitrogen was used to displace the air in the reactor, hydrogen was used to displace the nitrogen twice. Under a pressure of 0-0.05 MPa and a temperature maintained at 5-15 °C, hydrogen gas was introduced for 12 hours. The reaction completion was confirmed by HPLC analysis. The catalyst was then filtered off (recoverable and reusable), and the filtrate was subjected to reduced-pressure solvent removal to obtain 43g of an oily liquid, identified as 2-fluoro-3-aminobenzoate methyl ester with an HPLC purity of 97% and a yield of 98.3%. This product was used for subsequent synthesis.

### Synthetic Preparation Example 4

In a 500mL hydrogenation reactor, 250g of methanol and 2g of 5% palladium on carbon (dry basis, 50% moisture content) were charged. Then, 52g of the solid ethyl 2-fluoro-3-nitrobenzoate obtained from Synthetic Preparation Example 2 was added. Then the reactor was sealed. After nitrogen was used to displace the air in the reactor, hydrogen was used to displace the nitrogen twice. Under a pressure of 0-0.05 MPa and a temperature maintained at 5-15 °C, hydrogen gas was introduced for 12 hours. The reaction completion was confirmed by HPLC analysis. The catalyst was then filtered off (recoverable and reusable), and the filtrate was subjected to reduced-pressure solvent removal to obtain 46g of an oily liquid, identified as ethyl 2-fluoro-3-aminobenzoate with an HPLC area-normalized content of 96% and a yield of 96.3%. This product was used for subsequent synthesis.

### Synthetic Preparation Example 5

25g (0.20mol) of benzoic acid and 72g (0.60mol) of thionyl chloride were added into a reaction vessel. The mixture was heated under reflux for 2-5 hours. Then the reaction mixture was cooled to room temperature. The solvent was removed from the reaction solution by rotary evaporation under reduced pressure until the temperature reached 80°C to obtain an oily liquid of benzoyl chloride. The oily liquid was cooled to about 25°C, and then 25g of freshly prepared dichloroethane was added to dissolve it for later use.

In another 250ml four-neck glass reaction flask, 100g of dichloroethane, 35g (0.20mol) of methyl 3-amino-2-fluorobenzoate, and 24g (0.30mol) of pyridine were added. The reaction flask was cooled in an ice bath to a temperature of 5-25 °C. The previously prepared benzoyl chloride solution was added dropwise within 1 hour. The reaction was carried out at a temperature of 5-25°C for 1-2 hours. After the reaction was completed, 50ml of water was added, and the mixture was stirred for 0.5 hours. Then, the layers were separated, and the solvent layer was transferred to a 500ml rotary evaporator. The solvent was removed under reduced pressure until the temperature reached 80°C, and 53g of off-white solid methyl 2-fluoro-3-[(phenylcarbonyl) amino] benzoate was obtained. The HPLC area-normalized content of the product is 94% (the yield is 91.2%). It was set aside without further treatment and directly used for the synthesis reaction.

### Synthetic Preparation Example 6

Following the similar operation in Synthetic Preparation Example 5, in which the raw material methyl 3-amino-2-fluoro-benzoate (35g, 0.20mol) was replaced with ethyl 3-amino-2-fluoro-benzoate (38g, 0.20mol) obtained from Synthetic Preparation Example 4, while other conditions remained unchanged. Finally, 55g of off-white solid ethyl 2-fluoro-3-[(phenylcarbonyl) amino] benzoate was obtained. The HPLC area-normalized content of the product was 94% (the yield was 90%). It was set aside without further treatment and directly used for the synthesis reaction.

### Synthetic Preparation Example 7

Following the operation in Synthetic Preparation Example 5, 25g (0.20mol) of benzoic acid was replaced with 28g (0.20mol) of p-fluoro benzoic acid, while keeping the other operations the same. Finally, 56g of off-white solid methyl 2-fluoro-3-[(4-fluorophenylcarbonyl) amino] benzoate was obtained. The HPLC area-normalized content of the product was 94% (the yield was 90.2%). It was set aside without further treatment and directly used for the synthesis reaction.

### Synthetic Example 1

### Synthesis of 2-fluoro-3-[(2-methoxyethyl)(phenylcarbonyl)amino]benzoic acid

In a 250ml four-neck glass reaction flask, 200g of the solvent N,N-dimethylformamide, 15g (0.10mol) of potassium carbonate (acting as a base), and 25g (0.086mol) of methyl 2-fluoro-3-[(phenylcarbonyl)amino]benzoate (synthesized in Synthetic Preparation Example 5) were added. Then, 1g of tetrabutylammonium bromide (used as a catalyst) was added. The mixture was heated to 55-65°C, and 15.8g (0.11mol) of 1-bromo-2-methoxyethane was added dropwise within 1 hour. The reaction was maintained at this temperature for about 2 hours. After that, the reaction mixture was cooled to room temperature with water. Then, it was filtered, and the filtrate was subjected to reduced-pressure solvent removal to obtain a residue. Subsequently, 50g of methanol and 60g (0.15mol) of 10% liquid alkali (sodium hydroxide solution) were added to the residue. The mixture was heated to 25-35 °C and maintained at this temperature for about 2 hours. Next, 150g of water was added, and 30% hydrochloric acid was used to adjust the pH to 1-2. Crystallization occurred at around 10-25 °C. The crystals were filtered, washed with water, and dried to obtain 26.5 g of off-white solid. The melting point of the solid was 155.8-157.9 °C. The HPLC area-normalized content was about 95%, and the yield was 92.3%. The product is 2-fluoro-3-[(2-methoxyethyl)(phenylcarbonyl)amino]benzoic acid. The reaction equation is as follows:

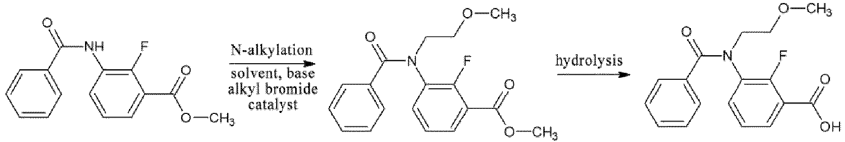

### Synthetic Example 2-4

Following the operation in Synthetic Preparation Example 1, 1g of tetrabutylammonium bromide as the catalyst was replaced respectively with 1g of tetrabutylammonium chloride, 1g of tetrabutylammonium hydrogen sulfate, and 1g of 18-crown-6 ether, while other feedstocks and operations remained unchanged. The obtained results were similar.

### Synthetic Example 5

In a 250ml four-neck glass reaction flask, 100g of the solvent toluene, 15g (0.10mol) of potassium carbonate (acting as a base), and 25g (0.086mol) of methyl 2-fluoro-3-[(phenylcarbonyl)amino]benzoate (synthesized in Synthetic Preparation Example 5) were added. Then, 1g of tetrabutylammonium bromide (used as a catalyst) and 15.8g (0.11mol) of 1-bromo-2-methoxyethane were added. A reflux water separator was installed, and the mixture was heated to reflux. The water generated during the reaction was removed through the reflux water separator. The reflux reaction lasted for about 2 hours until no more water was generated. After that, the reaction mixture was cooled to room temperature. Then, it was filtered, and the filtrate was subjected to reduced-pressure solvent removal to obtain a residue. Subsequently, 50g of methanol and 60g (0.15mol) of 10% liquid alkali were added to the residue. The mixture was heated to 25-35 °C and maintained at this temperature for about 2 hours. Next, 150g of water was added, and 30% hydrochloric acid was used to adjust the pH to 1-2. Crystallization occurred at around 10-25 °C. The crystals were filtered, washed with water, and dried to obtain 27g of off-white solid. The HPLC area-normalized content of the solid was about 95%, and the yield was 94%. The product was identified as 2-fluoro-3-[(2-methoxyethyl)(phenylcarbonyl)amino]benzoic acid.

### Synthetic Example 6-7

Following the operation in Synthetic Preparation Example 5, 100g of toluene as the solvent was replaced respectively with 100g of methylcyclohexane and 100g of xylene, while other feedstocks and operations remained unchanged. The obtained results were similar.

### Synthetic Example 8

### Synthesis of 2-fluoro-3-[(2-ethoxyethyl)(4-fluorophenylcarbonyl) amino] benzoic acid

In a 250ml four-neck glass reaction flask, 100g of dichloroethane as the solvent, 12.5g (0.088mol) of 4-fluoro benzoic acid, and 25g (0.20mol) of thionyl chloride were added. The mixture was heated under reflux for 2-5 hours. After that, the solvent was removed under reduced pressure to obtain an oily liquid, which is 4-fluoro benzoyl chloride and is set aside for later use.

In another 250ml four-neck glass reaction flask, 200g of the solvent N,N-dimethylformamide was added. Under nitrogen protection, 3.5g (0.091mol) of 60% sodium hydride as the base was added, followed by 15g (0.086mol) of methyl 2-fluoro-3-aminobenzoate synthesized in Synthetic Preparation Example 3, and 1g of tetrabutylammonium bromide as the catalyst. Then, 16.8g (0.11mol) of 1-bromo-2-ethoxyethane was added. The mixture was heated to 110-120°C and kept at this temperature for about 8 hours of reaction. After that, the reaction mixture was cooled to room temperature with water. After filtration, the filtrate was subjected to reduced-pressure solvent removal. The residue was extracted with 100ml of dichloroethane, and then washed with water. (The nuclear magnetic resonance hydrogen spectrum and mass spectrometry data of the product are attached below.) The solvent layer was dried with anhydrous magnesium sulfate. After filtration, at 25-35 °C, the obtained filtrate was added with 4-fluoro benzoyl chloride synthesized above and then heated to reflux for 2-5 hours of reaction. The solvent was removed under reduced pressure to obtain a residual oily material. (The nuclear magnetic resonance hydrogen spectrum and mass spectrometry data of the product are attached below.) Then, 50g of methanol and 60g (0.15mol) of 10% liquid alkali were added. The mixture was heated to 25-35°C and kept at this temperature for about 2 hours of reaction. After that, 150g of water was added, and 30% hydrochloric acid was used to adjust the pH to 1-2. Crystallization occurred at around 10-25 °C. The crystals were filtered, washed with water, and dried to obtain 29.4 g of pale yellow solid. The melting point of the solid was 77.3-79.2 °C. The HPLC area-normalized content was about 92%, and the yield was 90.1%. The product was 2-fluoro-3-[(2-ethoxyethyl)(phenylcarbonyl)amino]benzoic acid. (The nuclear magnetic resonance hydrogen spectrum and mass spectrometry data of the product are attached below.) The reaction equation is as follows:

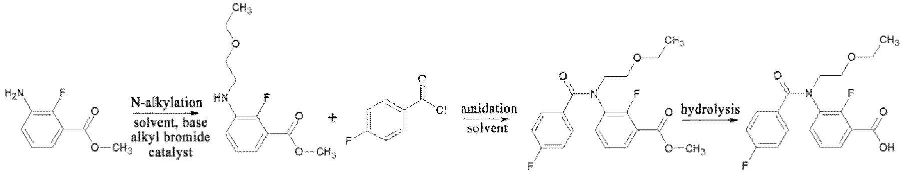

The nuclear magnetic resonance (NMR) hydrogen spectrum was measured using a Bruker AV-400 spectrometer (400MHz) with tetramethylsilane (TMS) as the internal standard. CDCl3 or DMSO-d6 was used as the solvent (the same below unless otherwise specified). The high-resolution mass spectrum was determined using a UHR-TOF maXis (ESI) mass spectrometer (the same below unless otherwise specified).

The nuclear magnetic resonance hydrogen spectrum and mass spectrometry data of methyl 2-fluoro-3-[(2-ethoxyethyl)amino]benzoate are as follows:
¹H-NMR (400 MHz, DMSO-d6) δ (ppm): 7.10-6.90 (m, 3H, ArH), 5.49 (s, 1H, NH), 3.80 (s, 3H, CH3O), 3.50 (t, J = 6.0 Hz, 2H, OCH2), 3.41 (q, J = 6.2 Hz, 2H, OCH2CH3), 3.25 (dd, J1 = J2 = 6.0 Hz, 2H, NCH2), 1.08 (t, J = 6.8 Hz, 3H, OCH2CH3).

HRMS (ESI) calcd. for C12H16FNNaO3 [(M+Na)+]: 264.1012; Found: 264.0988.

The nuclear magnetic resonance hydrogen spectrum and mass spectrometry data of methyl 2-fluoro-3-[(4-fluoro-N-(2-ethoxyethyl)benzoylamino]benzoate are as follows:
¹H-NMR (400 MHz, DMSO-d6) δ (ppm): 7.78-7.70 (m, 2H, ArH), 7.48-7.20 (m, 3H, ArH), 7.18-6.90 (m, 2H, ArH), 4.10-3.80 (m, 2H, OCH2), 3.75 (s, 3H, CH3O), 3.65-3.40 (m, 2H, NCH2), 3.28 (q, J = 6.2 Hz, 2H, OCH2CH3), 0.92 (t, J = 6.8 Hz, 3H, OCH2CH3).

HRMS (ESI) calcd. for C19H19F2NNaO4 [(M+Na)+]: 386.1180; Found: 386.1173.

The nuclear magnetic resonance hydrogen spectrum and mass spectrometry data of 2-fluoro-3-[(2-ethoxyethyl)(phenylcarbonyl)amino]benzoic acid are as follows:
¹H-NMR (400 MHz, DMSO-d6) δ (ppm): 10.20 (s, 1H, COOH), 7.75-7.60 (m, 2H, ArH), 7.38-7.15 (m, 3H, ArH), 7.12-6.90 (m, 2H, ArH), 4.10-3.82 (m, 2H, OCH2), 3.68-3.49 (m, 2H, NCH2), 3.30 (q, J = 6.2 Hz, 2H, OCH2CH3), 0.94 (t, J = 6.8 Hz, 3H, OCH2CH3).

HRMS (ESI) calcd. for C18H17F2NNaO4 [(M+Na)+]: 372.1023; Found: 372.1024.

### Synthetic Example 9

Following the operation in Synthetic Preparation Example 8, 1g of 4-dimethylaminopyridine (DMAP) was added during the second amidation step. Other feedstocks and operations remained unchanged. The obtained results were similar.

### Synthetic Example 10

### Synthesis of 2-fluoro-3-[(2-methoxyethyl)(4-fluorophenylcarbonyl) amino] benzoic acid

In a 250ml four-neck glass reaction flask, 100g of dichloroethane as the solvent, 12.5g (0.088mol) of p-fluoro benzoic acid, and 25g (0.20mol) of thionyl chloride were added. The mixture was heated under reflux for 2-5 hours. After that, the solvent was removed under reduced pressure to obtain an oily liquid, which is p-fluoro benzoyl chloride and is set aside for later use.

In another 250ml four-neck glass reaction flask, 100g of the solvent toluene was added, followed by 15g (0.10mol) of potassium carbonate (acting as a base), 15g (0.086mol) of methyl 2-fluoro-3-aminobenzoate synthesized in Synthetic Preparation Example 3, and 1g of tetrabutylammonium bromide (used as a catalyst). Then, 15.8g (0.11mol) of 1-bromo-2-methoxyethane was added. The mixture was heated to 100-120 °C and refluxed for dehydration reaction for about 5 hours. After that, the reaction mixture was cooled to room temperature with water. It was then filtered, washed with water, and subjected to reduced-pressure solvent removal to obtain a material (The nuclear magnetic resonance hydrogen spectrum and mass spectrometry data of the product are attached below).

The obtained material was dissolved in 100g of dichloroethane. At 25-35 °C, the p-fluoro benzoyl chloride synthesized above was added, and the mixture was heated to reflux and reacted for 2-5 hours. Then, the mixture was cooled, and 100ml of water was added. After layer separation and washing, the solvent was removed under reduced pressure to obtain a residual oily material (The nuclear magnetic resonance hydrogen spectrum and mass spectrometry data of the product are attached below). Subsequently, 50g of methanol and 60g (0.15mol) of 10% liquid alkali were added. The mixture was heated to 25-35 °C and kept at this temperature for about 2 hours of reaction. After that, 150g of water was added, and 30% hydrochloric acid was used to adjust the pH to 1-2. Crystallization occurred at around 10-25°C. The crystals were filtered, washed with water, and dried to obtain 28g of pale-yellow solid. The melting point of the solid was 120.5-122.5°C. The HPLC area-normalized content was about 93%, and the yield was 90.3%. The product is 2-fluoro-3-[(2-methoxyethyl)(4-fluorophenylcarbonyl)amino]benzoic acid (The nuclear magnetic resonance hydrogen spectrum and mass spectrometry data of the product are attached below). The reaction equation is as follows:

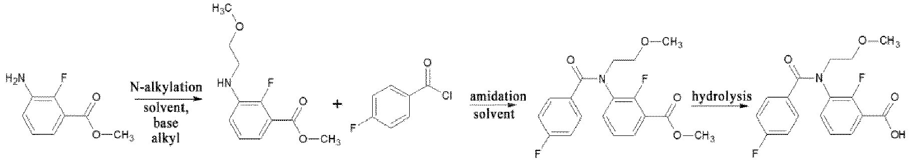

The nuclear magnetic resonance (NMR) hydrogen spectrum and mass spectrometry data of methyl 2-fluoro-3-[(2-methoxyethyl)amino]benzoate are as follows:
¹H-NMR (400 MHz, CDCl3) δ (ppm): 7.00-6.91 (m, 3H, ArH), 5.50 (s, 1H, NH), 3.80 (s, 3H, OCH3), 4.45 (t, J = 3.9 Hz, 2H, OCH2), 3.25-7.24 (m, 5H, NCH2 and OCH3).

HRMS (ESI) calcd. for C11H14FNNaO3 [(M+Na)+]: 250.0855; Found: 250.0836.

The nuclear magnetic resonance (NMR) hydrogen spectrum and mass spectrometry data of methyl 2-fluoro-3-(4-fluoro-N-(2-methoxyethyl)benzoylamino)benzoate are as follows:
1H-NMR (400 MHz, DMSO-d6) δ (ppm): 7.74-7.71 (m, 2H, ArH), 7.48-7.20 (m, 3H, ArH), 7.18-6.80 (m, 2H, ArH), 4.20-3.80 (m, 2H, OCH2), 3.79 (s, 3H, CH3O), 3.62-3.40 (m, 2H, NCH2), 3.13 (s, 3H, CH3O).

HRMS (ESI) calcd. for C18H17F2NNaO4 [(M+Na)+]: 372.1023; Found: 372.1024.

The nuclear magnetic resonance (NMR) hydrogen spectrum and mass spectrometry data of 2-fluoro-3-[(2-methoxyethyl)(4-fluorophenylcarbonyl) amino] benzoic acid are as follows:
¹H-NMR (400 MHz, DMSO-d6) δ (ppm): 10.32 (s, 1H, COOH), 7.70-7.60 (m, 2H, ArH), 7.38-7.20 (m, 3H, ArH), 7.15-6.90 (m, 2H, ArH), 4.15-3.82 (m, 2H, OCH2), 3.53-3.40 (m, 2H, NCH2), 3.14 (s, 3H, OCH3).

HRMS (ESI) calcd. for C17H15F2NNaO4 [(M+Na)+]: 358.0867; Found: 358.0858.

### Synthetic Example 11

### Synthesis of 2-fluoro-3-[(methyl)(phenylcarbonyl)amino]benzoic acid

In a 500ml pressure reactor, 200g of the solvent N,N-dimethylformamide, 15g (0.10mol) of potassium carbonate (a base), and 25g (0.086mol) of methyl 2-fluoro-3-[(phenylcarbonyl)amino]benzoate (synthesized in Synthetic Preparation Example 5) were added. Then, 1g of tetrabutylammonium bromide (a catalyst) was added. At room temperature, 20g (0.21mol) of methyl bromide was introduced into the reactor. After that, the reaction kettle was sealed. The mixture was heated to 55-65 °C and kept at this temperature for about 2 hours of reaction. Then it was cooled to room temperature, filtered, and the filtrate was subjected to reduced-pressure solvent removal to obtain an oily liquid. Subsequently, 50g of methanol and 60g (0.15mol) of 10% liquid alkali were added. The mixture was heated to 25-35 °C and maintained at this temperature for about 2 hours of reaction. After that, 150g of water was added, and 30% hydrochloric acid was used to adjust the pH to 1-2. Crystallization occurred at around 10-25 °C. The crystals were filtered, washed with water, and dried to obtain 23g of off-white solid. The HPLC area-normalized content of the solid was about 95%, and the yield was 93%. The product is 2-fluoro-3-[(methyl)(phenylcarbonyl)amino]benzoic acid. The reaction equation is as follows:

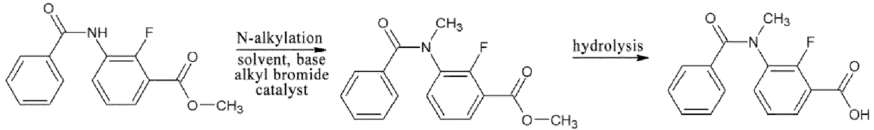

### Synthetic Example 12

Following the operation in Synthetic Preparation Example 11, 20g (0.21mol) of methyl bromide was replaced with 22g (0.20mol) of ethyl bromide and added to the pressure reactor. Other operations were similar. Finally, 24g of off-white solid was obtained. The HPLC area-normalized content was about 95%, and the yield was 92.3%. The product is 2-fluoro-3-[(ethyl)(phenylcarbonyl)amino]benzoic acid. The reaction equation is as follows:

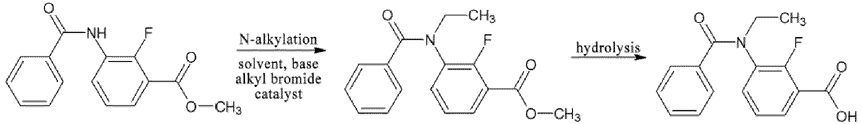

### Synthetic Example 13

In a 250ml four-neck glass reaction flask, 100g of the solvent toluene, 15g (0.10mol) of potassium carbonate (acting as a base), and 26.7g (0.086mol) of methyl 2-fluoro-3-[(4-fluorophenylcarbonyl)amino]benzoate (synthesized in Synthetic Preparation Example 7) were added. Then, 1g of tetrabutylammonium bromide (used as a catalyst) and 15.8g (0.11mol) of 1-bromo-2-methoxyethane were added. A reflux water separator was installed, and the mixture was heated to reflux. The water generated during the reaction was removed through the reflux water separator. The reflux reaction lasted for about 2 hours until no further water was generated. After that, the reaction mixture was cooled to room temperature. Then, it was filtered, and the filtrate was subjected to reduced-pressure solvent removal to obtain a residue. Subsequently, 50g of methanol and 60g (0.15mol) of 10% liquid alkali were added to the residue. The mixture was heated to 25-35 °C and maintained at this temperature for about 2 hours. Next, 150g of water was added, and 30% hydrochloric acid was used to adjust the pH to 1-2. Crystallization occurred at around 10-25 °C. The crystals were filtered, washed with water, and dried to obtain 28.5 g of off-yellow solid. The melting point of the solid was 122.5-123.4 °C. The HPLC area-normalized content was about 95%, and the yield was 93.9%. The product is 2-fluoro-3-[(2-methoxyethyl)(4-fluorophenylcarbonyl)amino]benzoic acid. The reaction equation and the nuclear magnetic resonance hydrogen spectrum and mass spectrometry data of the product are as follows:

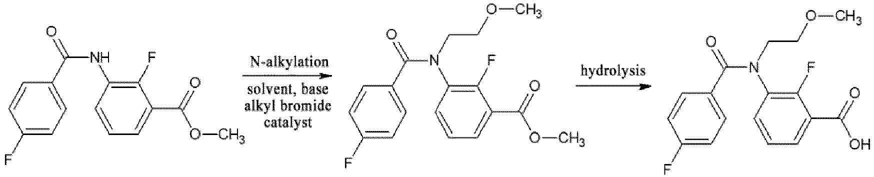

¹H-NMR (400 MHz, DMSO-d6) δ (ppm): 10.32 (s, 1H, COOH), 7.70-7.60 (m, 2H, ArH), 7.38-7.20 (m, 3H, ArH), 7.15-6.90 (m, 2H, ArH), 4.15-3.82 (m, 2H, OCH2), 3.53-3.40 (m, 2H, NCH2), 3.14 (s, 3H, OCH3).

HRMS (ESI) calcd. for C17H15F2NNaO4 [(M+Na)+]: 358.0867; Found: 358.0858.

### Synthetic Example 14

Following the operation in Synthetic Preparation Example 13, 15.8g (0.11mol) of 1-bromo-2-methoxyethane was replaced with 16.5g (0. 12mol) of bromomethyl cyclopropane, while other operations remained the same. Finally, 28g of off-white solid was obtained. The HPLC area-normalized content was about 95%, and the yield was 93.3%. The product is 2-fluoro-3-[(cyclopropylmethyl)(4-fluorophenylcarbonyl)amino]benzoic acid.

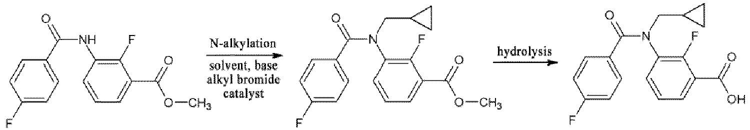

### Synthetic Example 15-16

Following the operations in Synthetic Preparation Example 1 and Synthetic Preparation Example 5 respectively, 25g (0.086mol) of methyl 2-fluoro-3-[(phenylcarbonyl)amino]benzoate was replaced with 26.3g (0.086mol) of ethyl 2-fluoro-3-[(phenylcarbonyl)amino]benzoate obtained in Synthetic Preparation Example 6. Other operations remained the same, and the obtained results were similar.

### Comparative Synthetic Example 1

Following the operation in Synthetic Preparation Example 1, 1g of tetrabutylammonium bromide (the catalyst) was not added. Other feedstocks and operations remained the same. Finally, 24g of off-white solid was obtained. The HPLC area-normalized content of the target product, 2-fluoro-3-[(2-methoxyethyl)(phenylcarbonyl)amino]benzoic acid, was about 55%, and the yield was 48.3%. The main by-product was 2-fluoro-3-[(phenylcarbonyl)amino]benzoic acid.

### Comparative Synthetic Example 2

Following the operation in Synthetic Preparation Example 5, 1g of tetrabutylammonium bromide (the catalyst) was not added. Other feedstocks and operations remained the same. 21g of off-white solid was obtained. The HPLC area-normalized content of the target product, 2-fluoro-3-[(2-methoxyethyl)(phenylcarbonyl)amino]benzoic acid, was about 5%, and the yield was 3.8%. The main by-product was 2-fluoro-3-[(phenylcarbonyl)amino]benzoic acid.

### Comparative Synthetic Example 3

Following the operation in Synthetic Preparation Example 8, 1g of tetrabutylammonium bromide (the catalyst) was not added. Other feedstocks and operations remained unchanged. Finally, an oily material with an area-normalized content of about 45% was obtained, and no solid crystals of the target product were formed.

### Synthetic Example 17

### Synthesis and preparation of 2-fluoro-3-[(phenylcarbonyl)(2-methoxyethyl)amino]-N-[2-bromo-4-(1,1,1,2,3,3,3-heptafluoropropan-2-yl)-6-(trifluoromethyl)phenyl]benzamide

In a 250ml four-neck glass reaction flask, 200g of dichloroethane, 23.7g (0.071mol) of 2-fluoro-3-[(2-methoxyethyl)(phenylcarbonyl)amino]benzoic acid (obtained in Synthetic Preparation Example 1), and 24g (0.20mol) of thionyl chloride were added. The mixture was heated under reflux for 3-5 hours. Then it was cooled to room temperature, and the reaction solution was transferred to a 500ml rotary evaporator. The solvent was removed under reduced pressure until the temperature reached 80 °C to obtain an oily liquid acyl chloride. After that, the temperature was lowered to about 25 °C, and 150g of acetonitrile was added for dissolution. The solution was transferred to the reaction flask, and 27.8g (0.08mol) of 4-(1,1,1,2,3,3,3-heptafluoropropan-2-yl)-2-(trifluoromethyl)aniline was added. The mixture was heated under reflux for 5-10 hours. Subsequently, the mixture was cooled to room temperature, and 20g of powdered potassium carbonate was added. Liquid bromine (13.5g) was added dropwise over about 2 hours at 25-45 °C, and then the reaction was kept at this temperature for another 2 hours. The reaction mixture was filtered to remove the inorganic salt. The filtrate was subjected to reduced-pressure solvent removal to obtain a residue. Then, 120g of ethanol was added, and the mixture was heated under reflux for 1 hour. After that, it was cooled for crystallization at 0-5 °C for 1 hour. The crystals were filtered, dried to obtain 42g of white crystalline powder solid, which is the target compound. The melting point of the solid is 179.5-180.5 °C. The HPLC area-normalized content is about 98%, and the total yield of the three-step reaction is 81.9%. The reaction equation is as follows:

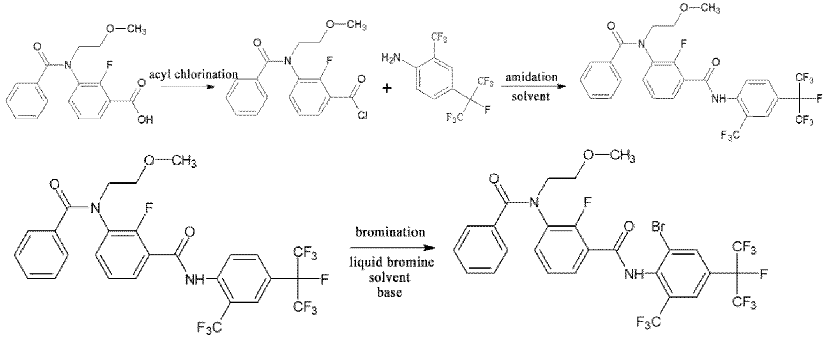

The nuclear magnetic resonance (NMR) hydrogen spectrum and mass spectrometry data are as follows:
The nuclear magnetic resonance (NMR) hydrogen spectrum was measured using a Bruker AV-400 spectrometer (400MHz) with tetramethylsilane (TMS) as the internal standard. DMSO-d6 was used as the solvent (the same below unless otherwise specified). The high-resolution mass spectrum was determined using a UHR-TOF maXis (ESI) mass spectrometer (the same below unless otherwise specified).

¹H-NMR (400 MHz, DMSO-d6) δ (ppm): 10.57 (s, 1H, CONH), 8.39 (s, 1H), 7.92 (s, 1H), 7.61-7.51 (m, 3H), 7.34-7.24 (m, 5H), 4.32-3.70 (m, 2H), 3.68-3.45 (m, 2H), 3.19 (s, 3H).

HRMS (ESI) calcd. for C₂₇H₁₈BrF₁₁N₂NaO₃ [(M+Na)⁺]: 729.0223 [(M+Na)⁺], 731.0202 [(M+2+Na)⁺]; Found: 729.0213 [(M+Na)⁺], 731.0196 [(M+2+Na)⁺].

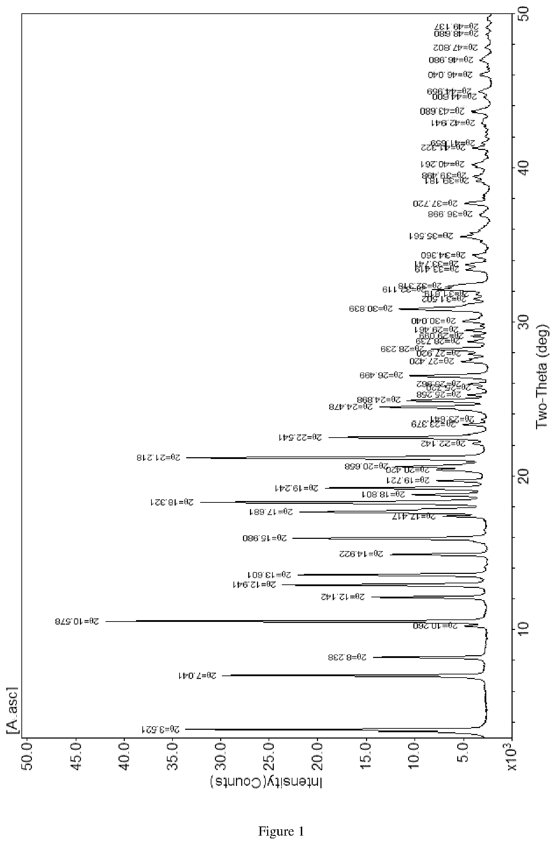

The target compound sample was analyzed by X-ray powder diffraction. The instrument used was the Rigaku SmartLab diffractometer (Japan) (the same below if not specifically stated). The crystalline substance has characteristic peaks at the following positions: 2θ = 3.521 ± 0.2°, 7.041 ± 0.2°, 8.238 ± 0.2°, 10.260 ± 0.2°, 10.578 ± 0.2°, 12.142 ± 0.2°, 12.941 ± 0.2°, 13.601 ± 0.2°, 14.922 ± 0.2°, 15.980 ± 0.2°, 17.417 ± 0.2°, 17.681 ± 0.2°, 18.321 ± 0.2°, 18.801 ± 0.2°, 19.241 ± 0.2°, 19.721 ± 0.2°, 20.420 ± 0.2°, 20.658 ± 0.2°, 21.218 ± 0.2°, 22.541 ± 0.2°, 23.379 ± 0.2°, 24.478 ± 0.2°, 24.898 ± 0.2°, 26.499 ± 0.2°, 27.420 ± 0.2°, 28.239 ± 0.2°, 29.461 ± 0.2°, 30.040 ± 0.2°, 30.839 ± 0.2°, 32.119 ± 0.2°, 32.318 ± 0.2°, 33.419 ± 0.2°, 33.741 ± 0.2°, 35.561 ± 0.2°, 37.720 ± 0.2°. For details, see Figure 1 in the appendix.

The sample obtained from this synthetic example was recrystallized with three times its weight of isopropyl ether. The final product was analyzed by X-ray powder diffraction, and the characteristic peaks of the crystalline form were found to be completely consistent with those described above.

The sample obtained from this synthesis example was recrystallized with three times its weight of ethylene glycol monomethyl ether. After that, the final obtained sample was analyzed by X-ray powder diffraction, and the characteristic peaks of the crystalline substance were the same as those mentioned above.

This indicates that the crystals of the compound obtained by crystallization from ethanol, isopropyl ether, and ethylene glycol monomethyl ether solvents respectively are of the same crystal type, easy to obtain, and have good stability.

### Synthetic Example 18

### Synthesis and preparation of 2-fluoro-3-[(4-fluorophenylcarbonyl)(2-methoxyethyl)amino]-N-[2-bromo-4-(1,1,1,2,3,3,3-heptafluoropropan-2-yl)-6-(trifluoromethyl)phenyl]benzamide

In a 250ml four-neck glass reaction flask, 200g of dichloroethane, 25g (0.071mol) of 2-fluoro-3-[(2-methoxyethyl)(4-fluorophenylcarbonyl)amino]benzoic acid (obtained in Synthetic Preparation Example 13), and 24g (0.20mol) of thionyl chloride were added. The mixture was heated under reflux for 3-5 hours. Then it was cooled to room temperature, and the reaction solution was transferred to a 500ml rotary evaporator. The solvent was removed under reduced pressure until the temperature reached 80 °C to obtain an oily liquid acyl chloride.

After that, the temperature was lowered to about 25 °C, and 150g of fresh dichloroethane was added for dissolution. The solution was transferred to the reaction flask, and 27.8g (0.08mol) of 4-(1,1,1,2,3,3,3-heptafluoropropan-2-yl)-2-(trifluoromethyl)aniline was added. The mixture was heated under reflux for 5-10 hours. Subsequently, the mixture was cooled to room temperature, and 5g of sodium hydroxide was added. Bromine (13.5g) was added dropwise over about 2 hours at 35-55 °C, and then the reaction was kept at this temperature for another 2 hours. Then 50ml of water was added, and the layers were separated to remove the aqueous layer. The solvent layer was subjected to reduced-pressure solvent removal to obtain a residue. Then, 120g of ethylene glycol monomethyl ether was added, and the mixture was heated under reflux for 1 hour. After that, it was cooled for crystallization at 0-5 °C for 1 hour. The crystals were filtered, dried to obtain 44g of white crystalline powder solid, which is the target compound. The melting point of the solid is 152.4-153.2 °C. The HPLC area-normalized content is about 98%, and the total yield of the three-step reaction is 83.7%. The reaction equation is as follows:

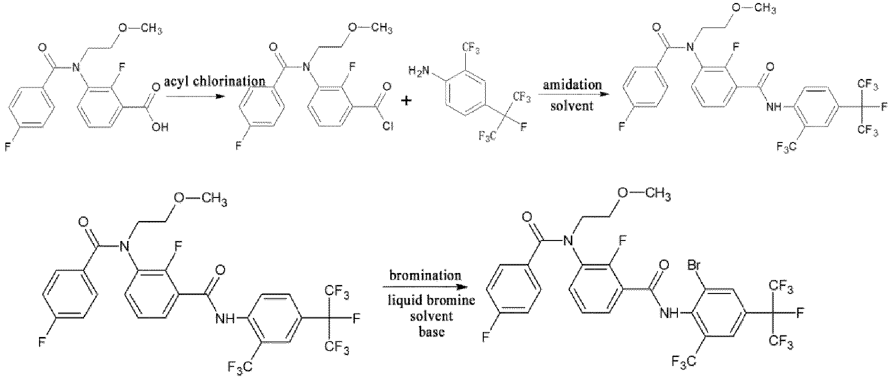

The nuclear magnetic resonance (NMR) hydrogen spectrum and mass spectrometry data are as follows:
¹H-NMR (400 MHz, DMSO-d6) δ (ppm): 10.56 (s, 1H, CONH), 8.38 (s, 1H), 7.92 (s, 1H), 7.65 (dd, J = 7.6 Hz, and 6.8 Hz, 1H), 7.60-7.49 (m, 1H), 7.42-7.20 (m, 3H), 7.10-7.02 (m, 2H), 4.10-3.70 (m, 2H), 3.68-3.42 (m, 2H), 3.19 (s, 3H, CH3O).

HRMS (ESI) calcd. for C27H17BrF12N2NaO3 [(M+Na)+]: 747.0129 [(M+Na)+], 749.0108 [(M+2+Na)+]; Found: 747.0111 [(M+Na)+], 749.0094 [(M+2+Na)+].

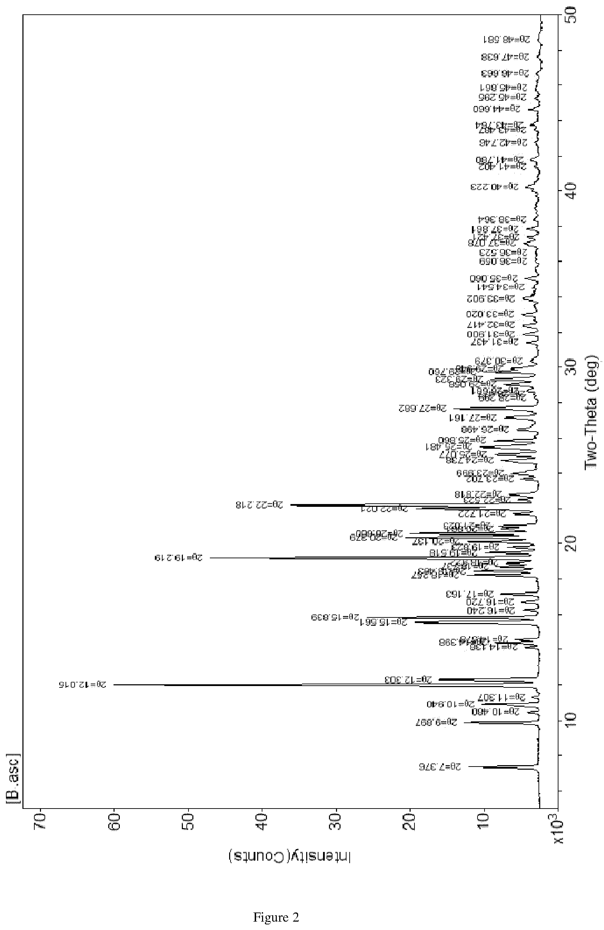

The target compound sample was analyzed by X-ray powder diffraction. The crystalline substance has characteristic peaks at the following positions: 2θ = 7.376 ± 0.2°, 9.897 ± 0.2°, 10.940 ± 0.2°, 12.015 ± 0.2°, 12.303 ± 0.2°, 14.398 ± 0.2°, 14.578 ± 0.2°, 15.561 ± 0.2°, 15.839 ± 0.2°, 17.163 ± 0.2°, 18.257 ± 0.2°, 18.463 ± 0.2°, 18.737 ± 0.2°, 18.922 ± 0.2°, 19.219 ± 0.2°, 19.518 ± 0.2°, 19.823 ± 0.2°, 20.137 ± 0.2°, 20.379 ± 0.2°, 20.600 ± 0.2°, 20.861 ± 0.2°, 21.023 ± 0.2°, 21.722 ± 0.2°, 22.021 ± 0.2°, 22.218 ± 0.2°, 22.818 ± 0.2°, 23.999 ± 0.2°, 24.738 ± 0.2°, 25.077 ± 0.2°, 25.481 ± 0.2°, 25.860 ± 0.2°, 27.161 ± 0.2°, 27.682 ± 0.2°, 29.058 ± 0.2°, 29.323 ± 0.2°, 29.760 ± 0.2°, 29.940 ± 0.2°. For details, see Figure 2 in the appendix.

The sample obtained from this synthesis example was recrystallized with three times its weight of ethanol. After that, the final obtained sample was analyzed by X-ray powder diffraction, and the characteristic peaks of the crystalline substance were the same as those mentioned above.

The sample obtained from this synthetic example was recrystallized with three times its weight of isopropyl ether.

The final product was analyzed by X-ray powder diffraction, and the characteristic peaks of the crystalline form were found to be completely consistent with those described above.

This indicates that the crystals of the compound obtained by crystallization from ethanol, isopropyl ether, and ethylene glycol monomethyl ether solvents respectively are of the same crystal type, easy to obtain, and have good stability.

### Synthetic Example 19

### Synthesis and preparation of 2-fluoro-3-[(4-fluorophenylcarbonyl)(2-methoxyethyl)amino]-N-[2-bromo-4-(1,1,1,2,3,3,3-heptafluoropropan-2-yl)-6-(trifluoromethyl)phenyl]benzamide

Following the operation in Synthetic Preparation Example 18, 25g (0.071mol) of 2-fluoro-3-[(2-methoxyethyl)(4-fluorophenylcarbonyl)amino]benzoic acid was replaced with 25.6g (0.071mol) of 2-fluoro-3-[(2-methoxyethyl)(4-fluorophenylcarbonyl)amino]benzoic acid obtained in Synthetic Preparation Example 10. Finally, 42g of white crystalline powder solid was obtained, which is the target compound. The melting point of the solid is 152.4-153.2 °C. The HPLC area-normalized content is about 98%, and the total yield of the three-step reaction is 79.9%. The sample was analyzed by NMR, mass spectrometry, and X-ray powder diffraction, and the results were completely consistent.

### Synthetic Example 20

### Synthesis and preparation of 2-fluoro-3-[(4-fluorophenylcarbonyl)(2-ethoxyethyl)amino]-N-[2-bromo-4-(1,1,1,2,3,3,3-heptafluoropropan-2-yl)-6-(trifluoromethyl)phenyl]benzamide

In a 250ml four-neck glass reaction flask, 200g of dichloroethane, 26g (0.071mol) of 2-fluoro-3-[(2-ethoxyethyl)(4-fluorophenylcarbonyl)amino]benzoic acid (obtained in Synthetic Preparation Example 8), and 24g (0.20mol) of thionyl chloride were added. The mixture was heated under reflux for 3-5 hours. Then it was cooled to room temperature, and the reaction solution was transferred to a 500ml rotary evaporator. The solvent was removed under reduced pressure until the temperature reached 80 °C to obtain an oily liquid acyl chloride.

After that, the temperature was lowered to about 25 °C, and 150g of toluene was added for dissolution. The solution was transferred to the reaction flask, and 27.8g (0.08mol) of 4-(1,1,1,2,3,3,3-heptafluoropropan-2-yl)-2-(trifluoromethyl)aniline was added. The mixture was heated under reflux for 5-10 hours. The solvent was removed under reduced pressure to obtain a residue. Then, 120g of methanol was added, and the mixture was cooled to room temperature. 5g of sodium hydroxide was added, and liquid bromine (13.5g) was added dropwise over about 2 hours at 35-55 °C. The reaction was kept at this temperature for another 2 hours. Then 50ml of water was added, and the layers were separated to remove the aqueous layer. The solvent layer was subjected to reduced-pressure solvent removal to obtain a residue. Then, 120g of isopropyl ether was added, and the mixture was heated under reflux for 1 hour. After that, it was cooled for crystallization at 0-5 °C for 1 hour. The crystals were filtered, dried to obtain 44.5g of white crystalline powder solid, which is the target compound. The melting point of the solid is 168.2-169.2 °C. The HPLC area-normalized content is about 98%, and the total yield of the three-step reaction is 83.1%. The reaction equation is as follows:

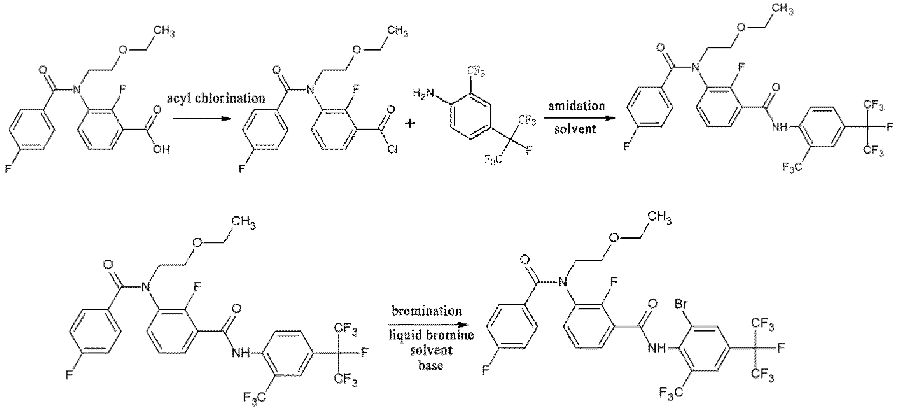

The nuclear magnetic resonance (NMR) hydrogen spectrum and mass spectrometry data are as follows:
¹H-NMR (400 MHz, DMSO-d6) δ (ppm): 10.52 (s, 1H, CONH), 8.38 (s, 1H), 7.92 (s, 1H), 7.67 (dd, J = 7.6 Hz, and 6.8 Hz, 1H), 7.60-7.50 (m, 1H), 7.48-7.20 (m, 3H), 7.18-6.95 (m, 2H), 4.20-3.72 (m, 2H), 3.68-3.45 (m, 2H), 3.42-3.32 (m, 2H), 1.01 (t, J = 6.8 Hz, 3H, CH3).

HRMS (ESI) calcd. For C₂₈H₁₉BrF₁₂N₂NaO₃ [(M+Na)⁺]: 761.0285 [(M+Na)⁺], 763.0265 [(M+2+Na)⁺]; Found: 761.0263 [(M+Na)⁺], 763.0248 [(M+2+Na)⁺].

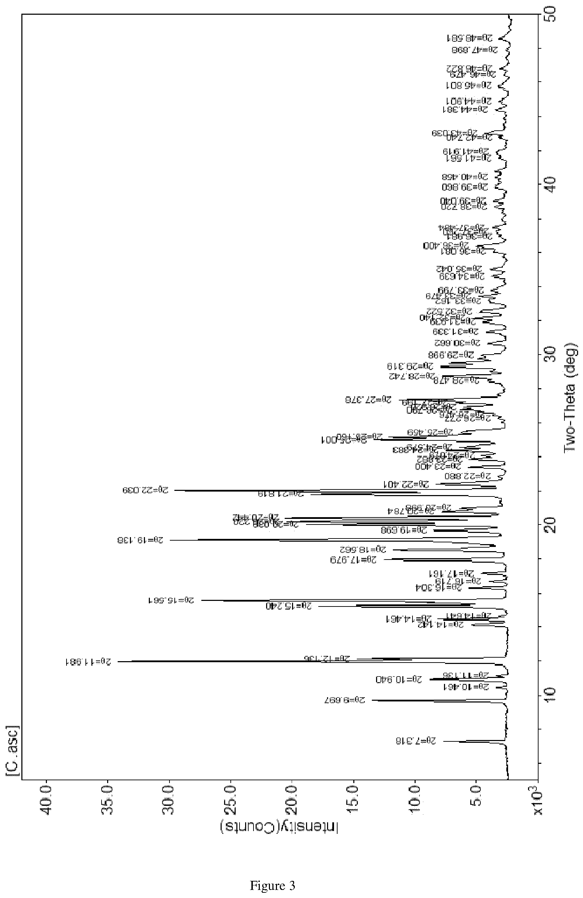

The target compound sample was analyzed by X-ray powder diffraction. The crystalline substance has characteristic peaks at the following positions: 2θ = 7.318 ± 0.2°, 9.697 ± 0.2°, 10.940 ± 0.2°, 11.981 ± 0.2°, 12.136 ± 0.2°, 14.142 ± 0.2°, 14.461 ± 0.2°, 15.240 ± 0.2°, 15.561 ± 0.2°, 16.304 ± 0.2°, 17.161 ± 0.2°, 17.979 ± 0.2°, 18.562 ± 0.2°, 19.138 ± 0.2°, 19.698 ± 0.2°, 20.039 ± 0.2°, 20.220 ± 0.2°, 20.442 ± 0.2°, 20.784 ± 0.2°, 20.998 ± 0.2°, 21.819 ± 0.2°, 22.039 ± 0.2°, 22.401 ± 0.2°, 23.400 ± 0.2°, 23.882 ± 0.2°, 24.383 ± 0.2°, 24.579 ± 0.2°, 25.001 ± 0.2°, 25.160 ± 0.2°, 25.459 ± 0.2°, 26.780 ± 0.2°, 26.920 ± 0.2°, 27.199 ± 0.2°, 27.378 ± 0.2°, 28.742 ± 0.2°, 29.319 ± 0.2°, 29.998 ± 0.2°, 31.339 ± 0.2°, 31.939 ± 0.2°, 32.140 ± 0.2°, 32.522 ± 0.2°, 33.479 ± 0.2°, 36.400 ± 0.2°, 43.039 ± 0.2°. For details, see Figure 3.

The sample obtained from this synthesis example was recrystallized with three times its weight of ethanol. The final obtained sample was analyzed by X-ray powder diffraction, and the characteristic peaks of the crystalline substance were the same as those mentioned above.

The sample obtained from this synthesis example was recrystallized with three times its weight of ethylene glycol monomethyl ether. The final obtained sample was analyzed by X-ray powder diffraction, and the characteristic peaks of the crystalline substance were exactly the same as those above.

The sample obtained from this synthesis example was recrystallized with three times its weight of ethylene glycol dimethyl ether. The final obtained sample was analyzed by X-ray powder diffraction, and the characteristic peaks of the crystalline substance were exactly the same as those above.

This indicates that the crystals of the compound obtained by crystallization from ethanol, isopropyl ether, ethylene glycol monomethyl ether, and ethylene glycol dimethyl ether solvents respectively are of the same crystal type, easy to obtain, and have good stability.

### Synthetic Example 21

### Synthesis and preparation of 2-fluoro-3-[(phenylcarbonyl)(methyl)amino]-N-[2-bromo-4-(1,1,1,2,3,3,3-heptafluoropropan-2-yl)-6-(trifluoromethyl)phenyl]benzamide

Following the operation in Synthetic Preparation Example 18, 25g (0.071mol) of 2-fluoro-3-[(2-methoxyethyl)(4-fluorophenylcarbonyl)amino]benzoic acid as the raw material was replaced with 20.4g (0.071mol) of 2-fluoro-3-[(methyl)(phenyl carbonyl)amino]benzoic acid obtained in Synthetic Preparation Example 11. Other operations remained the same. Finally, 40.1g of white crystalline powder solid was obtained, which is the target compound. The melting point of the solid is 155.1-156.4 °C. The HPLC area-normalized content is about 98%, and the total yield of the three-step reaction is 83.4%. The reaction equation is as follows:

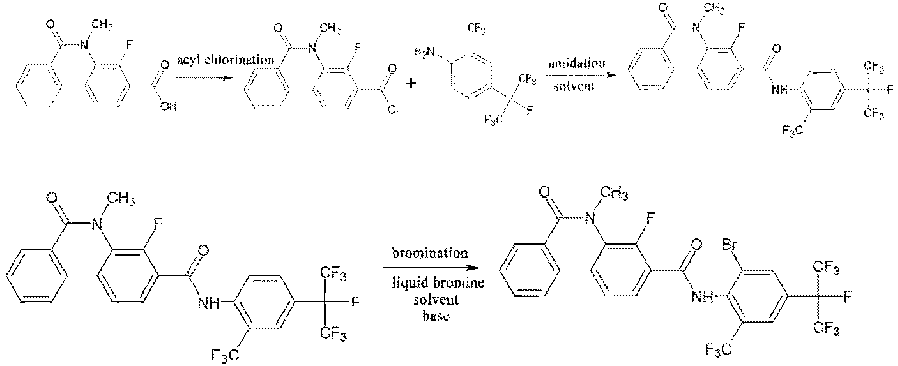

The nuclear magnetic resonance (NMR) hydrogen spectrum and mass spectrometry data are as follows:
¹H-NMR (400 MHz, DMSO-d6)δ(ppm): 10.65 (s, 1H, CONH), 8.39 (s, 1H), 7.93 (s, 1H), 7.61-7.51 (m, 2H), 7.45-7.15 (m, 6H), 3.32 (s, 3H).

HRMS (ESI) calcd. for C25H14BrF11N2NaO2 [(M+Na)+]: 684.9961 [(M+Na)+], 686.9940 [(M+2+Na)+]; Found: 684.9956 [(M+Na)+], 686.9940 [(M+2+Na)+].

### Synthetic Example 22

### Synthesis and preparation of 2-fluoro-3-[(phenylcarbonyl)(ethyl)amino]-N-[2-bromo-4-(1,1,1,2,3,3,3-heptafluoropropan-2-yl)-6-(trifluoromethyl)phenyl]benzamide

Following the operation in Synthetic Preparation Example 18, 25g (0.071mol) of 2-fluoro-3-[(2-methoxyethyl)(4-fluorophenylcarbonyl)amino]benzoic acid as the raw material was replaced with 21.4g (0.071mol) of 2-fluoro-3-[(ethyl)(phenyl carbonyl)amino]benzoic acid obtained in Synthetic Preparation Example 12. Other operations remained the same. 40g of white crystalline powder solid was obtained, which is the target compound. The melting point of the solid is 166.1-167.8 °C. The HPLC area-normalized content is about 98%, and the total yield of the three-step reaction is 81.5%. The reaction equation is as follows:

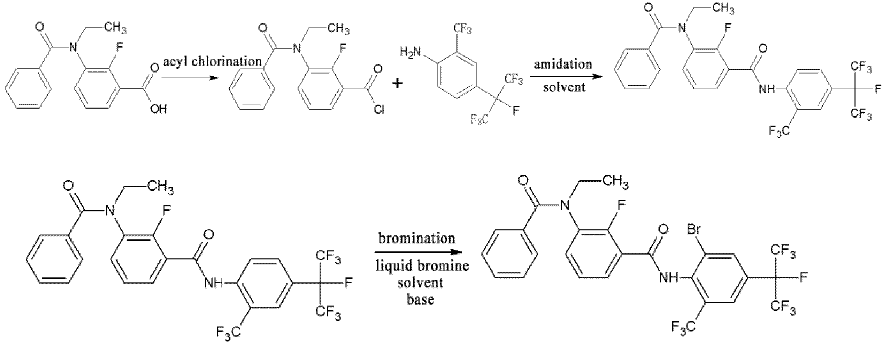

### Synthetic Example 23

### Synthesis and preparation of 2-fluoro-3-[(4-fluorophenylcarbonyl)(cyclopropylmethyl)amino]-N-[2-bromo-4-(1,1,1,2,3,3,3-heptafluoropropan-2-yl)-6-(trifluoromethyl)phenyl]benzamide

Following the operation in Synthetic Preparation Example 18, 25g (0.071mol) of 2-fluoro-3-[(2-methoxyethyl)(4-fluorophenylcarbonyl)amino]benzoic acid as the raw material was replaced with 23.4g (0.071mol) of 2-fluoro-3-[(cyclopropylmethyl)(4-fluorophenylcarbonyl)amino]benzoic acid obtained in Synthetic Preparation Example 14. Other operations remained the same. Finally, 43g of white crystalline powder solid was obtained, which is the target compound. The melting point of the solid is 152.5-153.3 °C. The HPLC area-normalized content is about 98%, and the total yield of the three-step reaction is 82.3%. The reaction equation is as follows:

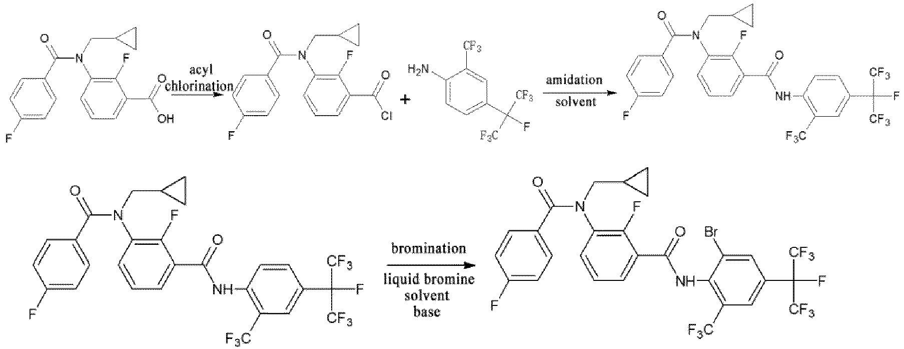

### Synthetic Example 24

### Synthesis and preparation of 2-fluoro-3-[(4-cyanophenylcarbonyl)(2-ethoxyethyl)amino]-N-[2-bromo-4-(1,1,1,2,3,3,3-heptafluoropropan-2-yl)-6-(trifluoromethyl)phenyl]benzamide

Following the operation in Synthetic Preparation Example 18, 25g (0.071mol) of 2-fluoro-3-[(2-methoxyethyl)(4-fluorophenylcarbonyl)amino]benzoic acid as the raw material was replaced with 26.6g (0.071mol) of 2-fluoro-3-[(2-ethoxyethyl)(4-cyanophenylcarbonyl)amino]benzoic acid obtained from the Synthetic Preparation Example 13. Other operations remained the same. Finally, 43.4g of off-white crystalline powder solid was obtained, which is the target compound. The melting point of the solid is 150.1-152.2 °C. The HPLC area-normalized content is about 98%, and the total yield of the three-step reaction is 80.3%. The reaction equation is as follows:

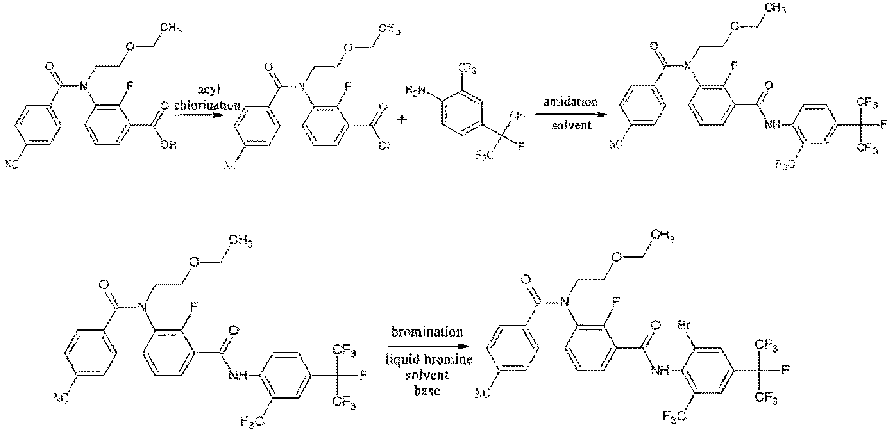

The nuclear magnetic resonance (NMR) hydrogen spectrum and mass spectrometry data are as follows:
¹H-NMR (400 MHz, DMSO-d6) δ (ppm): 10.48 (s, 1H, CONH), 8.39 (s, 1H), 7.92 (s, 1H), 7.72-7.65 (m, 3H), 7.58-7.50 (m, 1H), 7.48-7.35 (m, 2H), 7.32 (dd, J = 7.2 Hz, and 7.2 Hz, 1H), 4.15-3.85 (m, 2H), 3.70-3.50 (m, 2H), 3.44-3.32 (m, 2H), 1.00 (t, J = 6.0 Hz, 3H, CH3).

HRMS (ESI) calcd. for C29H19BrF11N3NaO3 [(M+Na)+]: 768.0332 [(M+Na)+], 770.0311 [(M+2+Na)+]; Found: 768.0310 [(M+Na)+], 770.0293 [(M+2+Na)+].

Other compounds of General Formula (I) or (II) according to the present invention can be synthesized by referring to the methods described in the above examples.

### Bioactivity Testing Examples

Several compounds of General Formula (II) obtained in the present invention were tested against various pests.

Compound Preparation: A certain mass of technical active compound was weighed with a balance (precision: 0.001g) and dissolved in DMF to prepare a 1% stock solution. The stock solution was then diluted with distilled water containing 0.1% Tween-80 to achieve the test concentration for later use. For formulated products, a specific mass of the formulation sample was weighed with a balance (precision: 0.001g) based on its active ingredient content, and diluted with distilled water to the required test concentration for later use.

**Table 1 Compounds of General Formula (II)**

| Compound | R¹ | R² | Melting point (°C) |
|---|---|---|---|
| SYN101 | H | methyl | 155.1-156.4 |
| SYN102 | H | ethyl | 166.1-167.8 |
| SYN103 | H | 2-methoxyethyl | 179.5-180.5 |
| SYN104 | F | cyclopropylmethyl | 152.5-153.3 |
| SYN105 | F | 2-methoxyethyl | 152.5-152.8 |
| SYN106 | F | 2-ethoxyethyl | 168.2-169.2 |

### Test Example 1

Determination of the Indoor Bioactivity of Some Compounds of General Formula (II) Against *Plutella xylostella* The susceptible and chlorantraniliprole-resistant strains of *Plutella xylostella* were reared indoors on radish seedlings. Test Method: Activity determination of *Plutella xylostella*

The leaf-dip method was used. An appropriate amount of corn leaves was immersed in the test solution for 30 seconds, then placed in plastic culture dishes lined with filter paper and allowed to air-dry naturally. Ten 2-instar larvae of *Plutella xylostella* were introduced into each dish. The dishes were placed in an observation room with a temperature of 22 °C and a photoperiod of 16 h light/8 h dark. After 2 days, the larvae were observed. Larvae unresponsive to gentle prodding with a brush were classified as dead. The experiment was repeated three times, and a blank control without test compound was included.

Test Results: The activity determination results of some compounds of General Formula (II) such as SYN101 against *Plutella xylostella* are shown in Table 2.

The results show that at the tested concentrations of 0.2 mg/L, 0.1 mg/L, and 0.05 mg/L, compounds SYN101, SYN102, SYN103, SYN104, SYN105, and SYN106 all exhibit excellent insecticidal activity (>80%) against both the susceptible and the chlorantraniliprole-resistant strains of *Plutella xylostella.* At the low concentration of 0.05 mg/L, SYN104, SYN105, and SYN106 still show excellent insecticidal activity against both the susceptible and the chlorantraniliprole-resistant strains of *Plutella xylostella,* reaching 100%.

**Table 2: Bioactivity Assay Results of Some Compounds of General Formula (II) Against Plutella xylostella**

| Compound | Concentration (mg/L) | Mortality (%) (Chlorantraniliprole-resistant Strain) | Mortality (%) (Susceptible Strain) |
|---|---|---|---|
| SYN101 | 0.2 | 100 | 100 |
| | 0.1 | 100 | 100 |
| | 0.05 | 86.67 | 83.33 |
| SYN102 | 0.2 | 100 | 100 |
| | 0.1 | 100 | 100 |
| | 0.05 | 83.33 | 80 |
| SYN103 | 0.2 | 100 | 100 |
| | 0.1 | 100 | 100 |
| | 0.05 | 93.33 | 86.67 |
| SYN104 | 0.2 | 100 | 100 |
| | 0.1 | 100 | 100 |
| | 0.05 | 100 | 100 |
| SYN105 | 0.2 | 100 | 100 |
| | 0.1 | 100 | 100 |
| | 0.05 | 100 | 100 |
| SYN106 | 0.2 | 100 | 100 |
| | 0.1 | 100 | 100 |
| | 0.05 | 100 | 100 |
| CK | | 0 | 0 |

### Test Example 2

Determination of the Indoor Bioactivity of Some Compounds of General Formula (II) Against *Spodoptera frugiperda* Activity determination of *Spodoptera frugiperda*: The leaf-dip method was used. An appropriate amount of corn leaves was immersed in the test solution for 30 seconds, then placed in plastic culture dishes lined with filter paper and allowed to air-dry naturally. Ten 2-instar larvae of *Spodoptera frugiperda* were introduced into each dish. The dishes were placed in an observation room with a temperature of 26 °C and a photoperiod of 16 h light/8 h dark. After 2 days, the larvae were observed. Larvae unresponsive to gentle prodding with a brush were classified as dead. The experiment was repeated three times, and a blank control without test compound was included.

Test Results: The indoor bioactivity determination test results of some compounds of General Formula (II) such as SYN101 against *Spodoptera frugiperda* are shown in Table 3.

The results show that compounds SYN101, SYN102, and SYN103 all exhibit good activity at the tested concentration of 0.2 mg/L, reaching 100%. At the tested concentration of 0.1 mg/L, their activity decreases slightly but remains above 80%. At the tested concentration of 0.05 mg/L, their activity is relatively poor. At the tested concentrations of 0.2 mg/L, 0.1 mg/L, and 0.05 mg/L, compounds SYN104, SYN105, and SYN106 all show excellent insecticidal activity, reaching 100%.

**Table 3: Bioactivity Assay Results of Some Compounds of General Formula (II) Against Spodoptera frugiperda**

| Compound | Concentration (mg/L) | Number of Dead Larvae | Mortality (%) |
|---|---|---|---|
| SYN101 | 0.2 | 30 | 100 |
| | 0.1 | 26 | 86.67 |
| | 0.05 | 13 | 43.33 |
| SYN102 | 0.2 | 30 | 100 |
| | 0.1 | 26 | 86.67 |
| | 0.05 | 12 | 40 |
| SYN103 | 0.2 | 30 | 100 |
| | 0.1 | 28 | 93.33 |
| | 0.05 | 15 | 50 |
| SYN104 | 0.2 | 30 | 100 |
| | 0.1 | 30 | 100 |
| | 0.05 | 30 | 100 |
| SYN105 | 0.2 | 30 | 100 |
| | 0.1 | 30 | 100 |
| | 0.05 | 30 | 100 |
| SYN106 | 0.2 | 30 | 100 |
| | 0.1 | 30 | 100 |
| | 0.05 | 30 | 100 |
| CK | | 0 | 0 |

The applicant of the present invention declares that the present invention is illustrated by the above representative examples for the method for preparing a bisamide compound containing a heptafluoroisopropyl group and its intermediates. However, the present invention is not limited to the above examples, nor does it mean that the present invention must rely on the above examples to be implemented. Those skilled in the art should understand that any improvement to the present invention, equivalent substitution of each raw material of the product of the present invention, addition of auxiliary components, selection of specific methods, etc., all fall within the scope of protection and the scope of disclosure of the present invention.

## Claims

1. A method for preparing an intermediate compound for a bisamide compound containing a heptafluoroisopropyl group, wherein the intermediate compound is represented by General Formula (I) below: 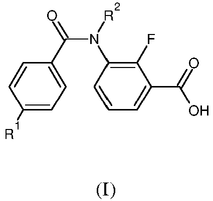 in which,
the substituent R1 is independently selected from H, fluorine, trifluoromethyl, cyano, or nitro groups;
the substituent R2 is independently selected from 2-methoxyethyl, 2-ethoxyethyl, 3-methoxypropyl, 3-ethoxypropyl, 2-n-propoxyethyl, 2-isopropoxyethyl, 2-n-butoxyethyl, cyclopropylmethyl, methyl, or ethyl groups;
**characterized in that** the process for preparing the intermediate compound of the General Formula (I) is as shown in Scheme (1) or Scheme (2): 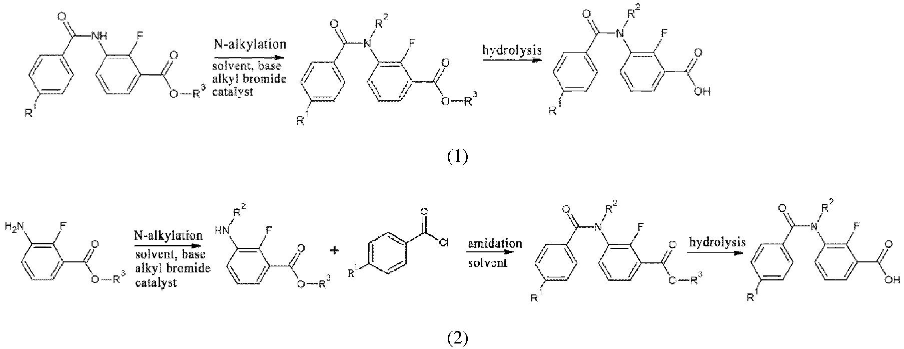 in which,
R3 is a C1-C5 alkyl group,
the solvent for the N-alkylation reaction is selected from N,N-dimethylformamide, N,N-dimethylacetamide, N,N-diethylformamide, N-methylpyrrolidone, 1,3-dimethyl-2-imidazolidinone, dimethyl sulfoxide, acetonitrile, tetrahydrofuran, dichloroethane, cyclohexane, methylcyclohexane, toluene, ethylbenzene, xylene, or mixtures thereof; the base for the N-alkylation reaction is selected from potassium carbonate, sodium carbonate, or sodium hydride; the alkyl bromide for the N-alkylation reaction is selected from 2-methoxybromoethane, 2-ethoxybromoethane, 3-methoxybromopropane, 3-ethoxybromopropane, bromomethylcyclopropane, bromomethane, or bromoethane;
the catalyst for the N-alkylation reaction is selected from cetyltrimethylammonium chloride, benzyltriethylammonium chloride, polyethylene glycol 400-800, tetrabutylammonium chloride, tetrabutylbromide, tetrabutylhydrogen sulfate, 18-crown-6 ether, or 4-dimethylaminopyridine (DMAP), or mixtures thereof;
the solvent for the amidation reaction is selected from N,N-dimethylformamide, N,N-dimethylacetamide, N,N-diethylformamide, N-methylpyrrolidone, 1,3-dimethyl-2-imidazolidinone, dimethyl sulfoxide, acetonitrile, tetrahydrofuran, ethyl acetate, dichloromethane, dichloroethane, cyclohexane, methylcyclohexane, toluene, ethylbenzene, xylene, or mixtures thereof.

2. A method for preparing a bisamide compound containing a heptafluoroisopropyl group, wherein the compound is represented by the structure of General Formula (II): 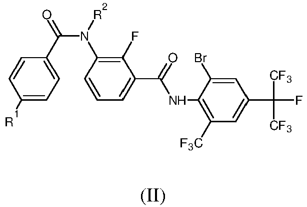 in which,
the substituent R1 is independently selected from H, fluorine, trifluoromethyl, cyano, or nitro groups;
the substituent R2 is independently selected from 2-methoxyethyl, 2-ethoxyethyl, 3-methoxypropyl, 3-ethoxypropyl, 2-n-propoxyethyl, 2-isopropoxyethyl, 2-n-butoxyethyl, cyclopropylmethyl, methyl, or ethyl groups;
**characterized in that** the process for preparing the compound represented by General Formula (II) is as shown in Scheme (3): 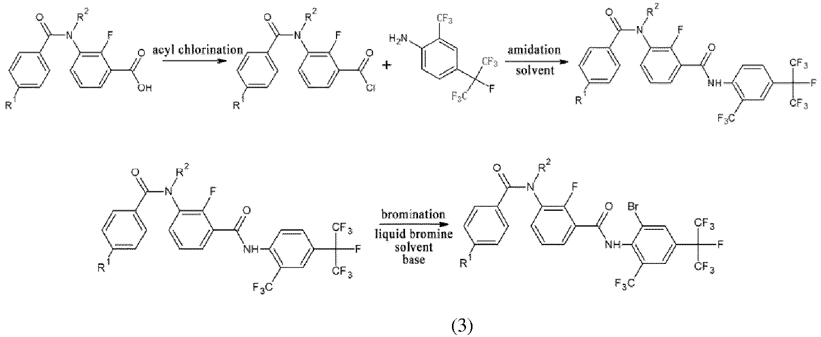 in which,
the substituent R1 is independently selected from H, fluorine, trifluoromethyl, cyano, or nitro groups;
the substituent R2 is independently selected from 2-methoxyethyl, 2-ethoxyethyl, 3-methoxypropyl, 3-ethoxypropyl, 2-n-propoxyethyl, 2-isopropoxyethyl, 2-n-butoxyethyl, cyclopropylmethyl, methyl, or ethyl groups;
the solvent for the amidation reaction is selected from dichloromethane, dichloroethane, cyclohexane, methylcyclohexane, toluene, ethylbenzene, xylene, ethyl acetate, tetrahydrofuran, or acetonitrile, or mixtures thereof; the solvent for the bromination reaction is selected from dichloromethane, dichloroethane, methanol, ethanol, isopropanol, tert-butanol, or mixtures thereof;
the base for the bromination reaction is selected from sodium hydroxide, potassium hydroxide, potassium carbonate, sodium carbonate, or their aqueous solutions.

3. The method according to claim 1, **characterized in that** R3 is methyl or ethyl.

4. The method according to claim 1, **characterized in that** R3 is methyl.

5. The method according to claim 1 or 2, **characterized in that** the substituent R1 is independently selected from H or fluorine; the substituent R2 is independently selected from 2-methoxyethyl, 2-ethoxyethyl, cyclopropylmethyl, methyl, or ethyl.

6. The method according to claim 1, **characterized in that** the catalyst for the N-alkylation reaction is selected from tetrabutylammonium chloride, tetrabutylbromide, tetrabutylammonium hydrogen sulfate, 18-crown-6 ether, or 4-dimethylaminopyridine (DMAP), or mixtures thereof.

7. The method according to claim 1, **characterized in that** the temperature for the N-alkylation reaction is 50°C to 150°C; the temperature for the amidation reaction is 0°C to 150°C.

8. The preparation method according to claim 2, **characterized in that** the temperature for the amidation reaction is 20°C to 180°C; the temperature for the bromination reaction is 0°C to 100°C.

9. The method according to claim 2 or 8, **characterized in that** the solvent for the amidation reaction is selected from dichloroethane, cyclohexane, methylcyclohexane, toluene, or acetonitrile, or mixtures thereof; the solvent for the bromination reaction is selected from dichloroethane, methanol, ethanol, isopropanol, tert-butanol, or mixtures thereof; the base for the bromination reaction is selected from sodium hydroxide, potassium carbonate, or their aqueous solutions.

10. A crystalline form of the compound prepared by the method according to claim 2, wherein the compound has the structure represented by General Formula (II), and wherein R1 is H and R2 is 2-methoxyethyl, **characterized in that** the crystalline form exhibits characteristic peaks in the X-ray powder diffraction at the following positions: 2θ = 3.521 ± 0.2°, 7.041 ± 0.2°, 8.238 ± 0.2°, 10.260 ± 0.2°, 10.578 ± 0.2°, 12.142 ± 0.2°, 12.941 ± 0.2°, 13.601 ± 0.2°, 14.922 ± 0.2°, 15.980 ± 0.2°, 17.417 ± 0.2°, 17.681 ± 0.2°, 18.321 ± 0.2°, 18.801 ± 0.2°, 19.241 ± 0.2°, 19.721 ± 0.2°, 20.420 ± 0.2°, 20.658 ± 0.2°, 21.218 ± 0.2°, 22.541 ± 0.2°, 23.379 ± 0.2°, 24.478 ± 0.2°, 24.898 ± 0.2°, 26.499 ± 0.2°, 27.420 ± 0.2°, 28.239 ± 0.2°, 29.461 ± 0.2°, 30.040 ± 0.2°, 30.839 ± 0.2°, 32.119 ± 0.2°, 32.318 ± 0.2°, 33.419 ± 0.2°, 33.741 ± 0.2°, 35.561 ± 0.2°, 37.720 ± 0.2°.

11. A crystalline form of the compound prepared by the method according to claim 2, wherein the compound has the structure represented by General Formula (II), and wherein R1 is fluorine and R2 is 2-methoxyethyl, **characterized in that** the crystalline form exhibits characteristic peaks in the X-ray powder diffraction at the following positions: 2θ = 7.376 ± 0.2°, 9.897 ± 0.2°, 10.940 ± 0.2°, 12.015 ± 0.2°, 12.303 ± 0.2°, 14.398 ± 0.2°, 14.578 ± 0.2°, 15.561 ± 0.2°, 15.839 ± 0.2°, 17.163 ± 0.2°, 18.257 ± 0.2°, 18.463 ± 0.2°, 18.737 ± 0.2°, 18.922 ± 0.2°, 19.219 ± 0.2°, 19.518 ± 0.2°, 19.823 ± 0.2°, 20.137 ± 0.2°, 20.379 ± 0.2°, 20.600 ± 0.2°, 20.861 ± 0.2°, 21.023 ± 0.2°, 21.722 ± 0.2°, 22.021 ± 0.2°, 22.218 ± 0.2°, 22.818 ± 0.2°, 23.999 ± 0.2°, 24.738 ± 0.2°, 25.077 ± 0.2°, 25.481 ± 0.2°, 25.860 ± 0.2°, 27.161 ± 0.2°, 27.682 ± 0.2°, 29.058 ± 0.2°, 29.323 ± 0.2°, 29.760 ± 0.2°, 29.940 ± 0.2°.

12. A crystalline form of the compounds prepared according to the preparation method of claim 2, wherein the compounds have the structure shown in the General Formula (II), when R1 is fluorine and R2 is 2-ethoxyethyl, **characterized in that** the crystalline form exhibits characteristic peaks in the X-ray powder diffraction at the following positions: 2θ = 7.318 ± 0.2°, 9.697 ± 0.2°, 10.940 ± 0.2°, 11.981 ± 0.2°, 12.136 ± 0.2°, 14.142 ± 0.2°, 14.461 ± 0.2°, 15.240 ± 0.2°, 15.561 ± 0.2°, 16.304 ± 0.2°, 17.161 ± 0.2°, 17.979 ± 0.2°, 18.562 ± 0.2°, 19.138 ± 0.2°, 19.698 ± 0.2°, 20.039 ± 0.2°, 20.220 ± 0.2°, 20.442 ± 0.2°, 20.784 ± 0.2°, 20.998 ± 0.2°, 21.819 ± 0.2°, 22.039 ± 0.2°, 22.401 ± 0.2°, 23.400 ± 0.2°, 23.882 ± 0.2°, 24.383 ± 0.2°, 24.579 ± 0.2°, 25.001 ± 0.2°, 25.160 ± 0.2°, 25.459 ± 0.2°, 26.780 ± 0.2°, 26.920 ± 0.2°, 27.199 ± 0.2°, 27.378 ± 0.2°, 28.742 ± 0.2°, 29.319 ± 0.2°, 29.998 ± 0.2°, 31.339 ± 0.2°, 31.939 ± 0.2°, 32.140 ± 0.2°, 32.522 ± 0.2°, 33.479 ± 0.2°, 36.400 ± 0.2°, 43.039 ± 0.2°.

13. The crystalline form according to any of claims 10-12, **characterized in that** the crystallization solvent is selected from methanol, ethanol, isopropanol, tetrahydrofuran, isopropyl ether, ethylene glycol monomethyl ether, ethylene glycol monoethyl ether, ethylene glycol dimethyl ether, ethylene glycol diethyl ether, or methyl tert-butyl ether, or mixtures thereof.
